# EUROPEAN PATENT SPECIFICATION

(11) **EP 1 848 732 B1**
(45) Date of publication and mention of the grant of the patent: **27.07.2016**
(21) Application number: 06719369.8
(22) Date of filing: 20.01.2006
(51) Int. Cl.: C07K 14/24

(54) **YERSINIA SPP. POLYPEPTIDES AND METHODS OF USE**
YERSINIA SPP. POLYPEPTIDE UND VERWENDUNGSVERFAHREN
POLYPEPTIDES YERSINIA SPP. ET LEURS PROCEDES D'UTILISATION

(30) Priority: 21.01.2005 US 646106 P
(43) Date of publication of application: 31.10.2007
(60) Divisional application: 11075020.5
(73) Proprietor: Epitopix, LLC, Willmar, MN 56201 (US)
(72) Inventor: EMERY, Daryll A., New London, MN 56273 (US); STRAUB, Darren E., New London, MN 56273 (US); WONDERLING, Laura, Minneapolis, MN 55412 (US)
(74) Representative: Potter Clarkson LLP
(86) International application number: PCT/US2006/002474
(87) International publication number: WO 2006/079076

(56) References cited:
- WO-A-95/21627
- WO-A-98/24912
- WO-A-02/053180
- WO-A-03/044047
- WO-A-2006/011060
- KOOI C ET AL: "Characterization of monoclonal antibodies to Yersinia enterocolitica iron-regulated proteins" CANADIAN JOURNAL OF MICROBIOLOGY 1995 CANADA, vol. 41, no. 7, 1995, pages 562-571, XP009069696 ISSN: 0008-4166
- HOICZYK E ET AL: "Structure and sequence analysis of Yersinia YadA and Moraxella UspAs reveal a novel class of adhesins" EMBO JOURNAL, OXFORD UNIVERSITY PRESS, SURREY, GB, vol. 19, no. 22, 15 November 2000 (2000-11-15), pages 5989-5999, XP002317588 ISSN: 0261-4189
- PARKHILL J ET AL: "Genome sequence of Yersinia pestis, the causative agent of plague" NATURE, NATURE PUBLISHING GROUP, LONDON, GB, vol. 413, no. 6855, 4 October 2001 (2001-10-04), pages 523-527, XP002240957 ISSN: 0028-0836
- WONDERLING ET AL.: "A Novel Subunit Vaccine Protects Mice Against Yersinia Infection" ABSTRACTS OF THE 8TH ANNUAL CONFERENCE ON VACCINE RESEARCH, BALTIMORE, MD, USA, MAY 9-11, 2005, [Online] 9 May 2005 (2005-05-09), XP002391065 Retrieved from the Internet: URL:http://www.nfid.org/conferences/vaccin e05/abstracts.pdf> [retrieved on 2006-07-19]

## Description

### BACKGROUND

There are three *Yersinia* species that are pathogenic to humans: *Y. pestis, Y. pseudotacberculosis,* and *Y. enterocolitica. Y. pestis* is the causative agent of plague, while *Y. pseudotuberculosis* and specific pathogenic serovars of *Y. enterocolitica* cause gastrointestinal illnesses. Other species of *Yersinia,* including *Y. rohdei, Y. aldovae, Y. bercovieri, Y. frederiksenii, Y. intermedia, Y. kristensenii,* and *Y. moolaretti*, are considered enterocolitica-like opportunist pathogens with the ability to cause diarrheal illness in susceptible individuals (Agbonlahor, J Clin Microbiol, 23, 891-6, (1986), Cafferkey, et al., J Hosp Infect, 24, 109-15, (1993), Loftus, et al., Dig Dis Sci, 47, 2805-10, (2002)). The *Yersinia* can also infect other animal species causing a range of illnesses. Most wild and domestic species of mammals are prone to infections with the enteropathogens *Y. enterocolitica* and *Y. pseduotuberculosis,* although most of these infections are subclinical and such animals usually serve only as asymptomatic carriers of the pathogens for transmission to humans (Fantasia, et al., J Clin Microbiol, 22, 314-5, (1985), Fantasia, et al., Vet Rec, 132, 532-4, (1993), Fukushima, et al., J Clin Microbiol, 18, 981-2, (1983), Kageyama, et al., J Med Primatol, 31, 129-35, (2002), Kato, et al., Appl Environ Microbiol, 49, 198-200, (1985), Poelma, et al., Acta Zool Pathol Antverp, 3-9, (1977), Shayegani, et al., Appl Environ Microbiol, 52, 420-4, (1986), Yanagawa, et al., Microbiol Immunol, 22, 643-6, (1978).). However, there are reports that the enteropathogenic *Yersinia* have been associated with diarrheal illness and general malaise in domestic animals such as sheep, cattle, goats, pigs, dogs, birds and farmed deer (Jerrett, I. V., et al., Aust Vet J, 67, 212-4, (1990), Slee, K. J., et al., Aust Vet J, 65, 271-5, (1988), Slee, K. J. and C. Button, Aust Vet J, 67, 396-8, (1990), Slee, K. J. and C. Button, Aust Vet J, 67, 320-2, (1990), Zheng, X. B., J Appl Bacteriol, 62, 521-5, (1987)). *Y*. *pestis* can cause disease in a variety of rodent species as well as nonhuman primates (Davis, K. J., et al., Arch Pathol Lab Med, 120, 156-63, (1996), Meyer, K. F., et al., J Infect Dis, 129, Suppl:S85-12, (1974). *Y. pestis* is also associated with potentially severe infections in domestic cats (Gasper, P. W., et al., J Med Entomol, 30, 20-6, (1993)) and a few cases of *Y. pestis* infection have been reported in dogs (Orloski, K. A. and M. Eidson, J Am Vet Med Assoc, 207, 316-8, (1995)). In addition, *Yersinia ruckeri* is a pathogen of fish, causing redmouth disease in salmonids (Furones, M. D., et al., Ann. Rev. Fish Dis., 3, 105-125, (1993)).

Plague is undoubtedly one of the most devastating acute infectious disease in the recorded history of man, estimated to have killed 100 to 200 million people worldwide (Perry, R. D. and J. D. Fetherston, Clin Microbiol Rev, 10, 35-66, (1997)). In recent years plague outbreaks have been relatively uncommon in the U.S. and other industrialized countries, although endemic foci exist in all continents except Australia. Worldwide surveys indicated 2000 to 5000 annual cases of plague reported in the last several years, although epidemiologists suspect that many human cases of plague are unreported. *Y. pseudotuberculosis* outbreaks are fairly rare, and have occurred primarily in Finland, Japan, and the former Soviet Union (Inoue, M., et al., Zentralbl Bakteriol Mikrobiol Hyg [B], 186, 504-511, (1988), Nuorti, J. P., et al., J Infect Dis, 189, 766-774, (2004), Rodina, L. V., et al., Zh Mikrobiol Epidemiol Immunobiol, 116-118, (1998), Toyokawa, Y., et al., Kansenshogaku Zasshi, 67, 36-44, (1993)). Most *Y. pseudotuberculosis* infections are assumed to be transmitted by the oral-fecal route; however, a vehicle of transmission has not been identified in many cases. In the United States, infections by *Y. enterocolitica* are more common than those with *Y. pseudotuberculosis,* and are typically associated with the consumption of contaminated pork products (Ray, S. M., et al., Clin Infect Dis, 38 Suppl 3, S181-189, (2004)). The incidence of human disease caused by the *Y. enterocolitica* in the U.S. is difficult to determine, simply because infections associated with this organism are typically self-limiting and insufficient detection techniques have limited the ability to correctly diagnose the causative agent. However, FoodNet surveillance for 1996-1999 estimated approximately 1 case of *Y. enterocolitica* infection per 100,000 in the United States (Ray, S. M., et al., Clin Infect Dis, 38 Suppl 3, S181-189, (2004)).

Plague is an infectious disease of animals and humans having both enzootic and epizootic components of transmission. The most naturally occurring means of transmission is from an infected rodent reservoir to fleas, which serve as natural vectors for transmission to humans. However, human-to-human transmission can also occur by direct contact or respiratory inhalation of contaminated droplets (Pneumonic form). Nevertheless, in natural infections *Y. pestis* typically enter humans by a subcutaneous route into the bloodstream, where they travel to the lymph nodes and begin to multiply. Clinical manifestations of plague include large swollen masses near the lymph nodes, referred to as bubos. Occasionally, *Y. pestis* multiplies rapidly in the bloodstream, inducing septicemia with an accompanying general malaise that includes fever, headache, chills, and occasionally gastrointestinal disturbances. These symptoms are often misdiagnosed early, and antibiotic therapy may therefore be administered too late for effective intervention. Septicemic infection by *Y. pestis* has a 50% fatality rate (Perry, R. D. and J. D. Fetherston, Clin Microbiol Rev, 10, 35-66, (1997)), and can lead to pulmonary infection. The pneumonic form of plague is extremely infectious by the aerosol route and is characterized by a rapid onset of disease and a mortality rate close to 100%. Therefore, although antibiotic therapies are available and effective if administered early, the rapid onset of pneumonic plague and the misdiagnosis of septicemic plague are major obstacles in treatment of the disease.

*Y. enterocolitica* and *Y. pseudotuberculosis* are considered enteropathogens since most human infections are transmitted by the fecal-oral route and are limited to the gastrointestinal tract. In a normal host, *Y. enterocolitica* causes a diarrheal illness, which may be accompanied by fever and lower quadrant pain that mimics appendicitis. *Y. pseudotuberculosis* typically does not cause diarrheal illness, and is more likely to cause mesenteric lymphadenitis which can be misdiagnosed as appendicitis. Following ingestion, both organisms attach to the intestinal lymphoid tissues and traverse the mucosal layer, where they can subsequently multiply in the mesenteric lymph nodes and migrate to the spleen and liver (Lian, C. J., et al., J Med Microbiol, 24, 219-226, (1987), Une, T., Microbiol Immunol, 21, 505-516, (1977)). *Y. pseudotuberculosis* and some serotypes of *Y. enterocolitica* can also spread to the vascular system and cause fatal cases of septicemia (Bottone, E. J., Clin. Microbiol. Rev., 10, 257-276, (1997), Lenz, T., et al., J Infect Dis, 150, 963, (1984)), although these more invasive infections are typically limited to susceptible individuals. *Y*. *enterocolitica* has also been associated with septicemia following blood transfusions; in these cases, the blood supply was contaminated with the organism, which can survive and grow at refrigeration temperatures (Natkin, J. B., KG, Clin Lab Med, 19, 523-536, (1999)). Furthermore, intestinal *Yersinia* infections can lead to delayed sequelae such as reactive arthritis and thyroiditis (Bottone, E. J., Clin. Microbiol. Rev., 10, 257-276, (1997), Gaston, J. S., et al., Arthritis Rheum., 42, 2239-2242, (1999), Taccetti, G., et al., Clin Exp Rheumatol, 12, 681-684, (1994)). Antibiotic therapy has not been demonstrated to reduce the severity or duration of gastrointestinal illness caused by these two pathogens (Hoogkamp-Korstanje, J., J Antimicrob Chemother, 20, 123, (1987), Pai, C. H., et al., J Pediatr, 104, 308-11, (1984)). However, a susceptible host is typically treated with antibiotics to prevent more serious clinical manifestations of disease. Septicemia caused by either of these enteropathogens is also generally treated with antibiotics, and such therapies are frequently successful against *Y. enterocolitica* (Gayraud, M., et al., Clin Infect Dis, 17, 405-10, (1993)). In contrast, antibiotic therapy has traditionally been less effective in patients where septicemia is caused by *Y. pseudotuberculosis,* and the mortality rate associated with *Y. pseudotuberculosis* septicemia is approximately 75% (Natkin, J. B., KG, Clin Lab Med, 19, 523-536, (1999)).

Although natural infection by *Y. pestis* is rare in this country, there is fear that the organism will become a bioterrorism agent. As a tool of deliberate mass infection, the *Y. pestis* organism is a prime candidate due to several characteristics. First, the organism is highly infectious when spread by aerosol, a convenient method of mass dissemination. Second, there is a high mortality rate associated with *Y*. *pestis* infection if left untreated, and the pneumonic form of plague is distinguished by a rapid onset of symptoms that may be recognized too late for an effective intervention. Finally, *Y. pestis* has a well-defined genetic system, thus antibioticresistant strains are relatively easy to engineer.

Several plague vaccines with varying levels of efficacy and safety have been investigated. One of the earliest vaccines consisted of killed whole cells (KWC) of *Y. pestis;* this type of vaccine was first used in the late 1890's and confers protection against the bubonic form of plague. However, there is evidence that KWC immunizations offer little protection against pneumonic plague (Cohen, R. J. and J. L. Stockard, JAMA, 202, 365-366, (1967), Meyer, K. F., Bull World Health Organ, 42, 653-666, (1970)), and an additional drawback to these vaccines is that multiple injections over several months are required for protective immunity. An attenuated strain of *Y. pestis,* strain EV76, has been studied as a live vaccine for plague. In mouse studies, this vaccine has been shown to protect against both subcutaneous and inhalation challenges and requires as few as one dose for protection (Russell, P., et al., Vaccine, 13, 1551-1556, (1995)). However, strain EV76 is not fully avirulent, causing death in approximately 1% of vaccinated mice (Russell, P., et al., Vaccine, 13, 1551-1556, (1995)). Interestingly, there have been several unsuccessful attempts to create an avirulent strain of *Y. pestis* suitable for use as a live vaccine (Titball, R. W. and E. D. Williamson, Vaccine, 19, 4175-4184, (2001)).

Subunit vaccines are considered to be the most promising type of vaccine for safe and effective prevention of plague, primarily because there is no fear of adverse effects in a human host. Several surface proteins associated with *Yersinia* virulence were tested for their immunogenicity; all of these proteins induced an antibody response but only the F1 capsule and the secreted V antigen elicited good protection against challenge (Titball, R. W. and E. D. Williamson, Vaccine, 19, 4175-4184, (2001)). Both F1 and V antigen provide protection as individual antigens in animal models, although the combination of the two antigens provides superior protection. Many recent studies have tested F1/V vaccines formulated with alternative adjuvants in an attempt to find the best delivery system for the F1 and V antigens (Alpar, H. O., et al., Adv. Drug Deliv. Rev., 51, 173-201, (2001), Eyles, J. E., et al., J Control Release, 63, 191-200, (2000), Jones, S. M., et al., Vaccine, 19, 358-366, (2001), Reddin, K. M., et al., Vaccine, 16, 761-767, (1998), Williamson, E. D., et al., Vaccine, 19, 566-571, (2000), Williamson, E. D., et al., Vaccine, 14, 1613-9, (1996)).

Other innovative strategies have used attenuated *Salmonella* strains as vaccine carriers for *Y. pestis* antigens. When a *Salmonella aroA* mutant expressing an F1/V fusion protein was used as a vaccine strain, 86% of mice survived a subsequent lethal challenge dose of *Y. pestis* (Leary, S. E., et al., Microb Pathog, 23, 167-179, (1997)). Similarly, a vaccine consisting of a DNA plasmid bearing a gene encoding truncated-F1 capsule provided 80 to 100% protection in different mouse strains (Grosfeld, H., et al., Infect Immun, 71, 374-383, (2003)). In addition, a group of investigators mapped the B- and T- cell epitopes of the F1 antigen and utilized the immunoreactive peptides in vaccine formulations (Sabhnani, L., et al., FEMS Immunol Med Microbiol, 38, 215-29, (2003)). Their results indicated that a mixture of epitopic peptides protected 83% of mice against a lethal dose of *Y. pestis.*

In contrast to the extensive search for protective plague vaccines, very little research efforts have been focused on preventing infections by the enteropathogenic *Yersinia* species. However, a few studies have demonstrated promising results. For example, attenuated *Y. enterocolitica* strains administered orally to mice displayed protective effects, reducing the bacterial load in the spleen and liver following oral challenge (Igwe, E. I., et al., Infect Immun, 67, 5500-5507, (1999)). However, these strains were engineered primarily as live oral vaccine carriers, and no further testing of these strains for prevention of yersiniosis has been reported. Two subunit vaccines were demonstrated as effective in animal models of infection. The first consisted of cellular extracts from *Y. enterocolitica* and was administered intranasally to mice. The immunized mice demonstrated enhanced clearance of an intranasal challenge dose of *Y. enterocolitica* from the lungs (Di Genaro, M. S., et al., Microbiol. Immunol., 42, 781-788, (1998)). A second subunit vaccine was formulated using a heat shock protein HSP60 from *Y. enterocolitica* adjuvanted with interleukin-12 (Noll, A. and AutenriethIb, Infect Immun, 64, 2955-2961, (1996)). Immunizations with this vaccine resulted in significantly fewer bacteria in mouse spleens following challenge, illustrating a protective effect. Additional work utilized a vaccine consisting of DNA encoding the *Y. enterocolitica* HSP60 in intramuscular immunizations in mice (Noll, A., et al., Eur J Immunol, 29, 986-996, (1999)). This study demonstrated that hsp60 mRNA was present in various host tissues following immunization, but protection against *Y. enterocolitica* challenge was limited to the spleen and no protection was observed in the intestinal mucosa.

The similarities and differences between the diseases caused by the pathogenic *Yersinia* species have been the focus of much research in the past decade. This is partly due to several observations that suggest the pathogenic *Yersinia* provide a useful model of pathogen evolution. First, DNA hybridization studies and recent genomic comparisons of fully sequenced *Y. pestis* and *Y. pseudotuberculosis* strains have indicated that these two pathogens are highly related (Chain, P. S., et al., Proc. Natl. Acad. Sci. USA, 101, 13826-13831, (2004), Ibrahim, A., et al., FEMS Microbiol Lett, 114, 173-177, (1993)), and it has been estimated that *Y. pestis* evolved from *Y. pseudotuberculosis* as recently as 1,500 to 20,000 years ago (Achtman, M., et al., Proc. Natl. Acad. Sci. USA, 96, 14043-14048, (1999)). However, despite their close evolutionary relationship, *Y*. *pseudotuberculosis* and *Y. pestis* cause very different diseases in humans. Furthermore, partial sequencing and 16s RNA hybridization studies suggested that *Y. enterocolitica* is more distantly related to the other pathogenic species of this genus (Ibrahim, A., et al., FEMS Microbiol Lett, 114, 173-177, (1993), Moore, R. L. and R. R. Brubaker, Int J Syst Bacteriol, 25, 336-339, (1975)), although *Y*. *enterocolitica* causes gastrointestinal infections similar to those observed with *Y. pseudotuberculosis.* Recent research has thus been focused on the virulence genes of the three pathogenic *Yersinia* species in an attempt to elucidate the different mechanisms they employ to cause disease. Mouse models have been particularly instructive in studying *Yersinia* pathogenesis, since all three species cause similar diseases in mice when injected intravenously, and more natural infections can be effectively simulated through oral and pneumonic challenge routes in mice.

A few virulence factors are unique to *Y. pestis.* These include proteins encoded on the *Y. pestis* plasmids pPCP and pMT, plasmids that are not found in *Y. enterocolitica* or *Y. pseudotuberculosis.* The pPCP plasmid encodes the plasminogen activator, a protein involved in rapid dissemination of bacteria into mammalian host tissues following subcutaneous injection (Sodeinde, O. A., et al., Science, 258, 1004-1007, (1992)). The pMT plasmid harbors at least two genes that aid in the infection of non-human hosts. The pMT-encoded *caf1* gene is required for assembly of the F1 capsule, a factor that inhibits phagocytosis in the murine host but is not required for virulence in primates (Friedlander, A. M., et al., Clin. Infect. Dis., 21 Suppl 2, S 178-181, (1995)). The murine toxin is also encoded on the pMT plasmid, and is believed to promote survival in the flea although it is not a required virulence factor in murine hosts (Hinnebusch, B. J., et al., Science, 296, 733-735, (2002), Hinnebusch, J., et al., Int J Med Microbiol, 290, 483-487, (2000)). Other differences between the species are the structures of the lipopolysaccharide (LPS) molecules produced by the yersiniae. Both *Y. enterocolitica* and *Y. pseudotuberculosis* express variable O-antigen side chains, which have been theorized to enhance survival in the gastrointestinal tract (Reeves, P., Trends Microbiol., 3, 381-386, (1995)) and may inhibit complement-mediated lysis during invasive disease (Karlyshev, A. V., et al., Infect Immun, 69, 7810-7819, (2001)). In contrast, *Y. pestis* has a rough LPS phenotype with no O-specific side chains due to mutations in several O-antigen biosynthesis genes (Prior, J. G., et al., Microb. Pathog., 30, 48-57, (2001), Skurnik, M. P., A; Ervela, E, Mol Microbiol, 37, 316-330, (2000)).

Interestingly, genomic sequencing projects revealed that several virulence genes present in all three pathogenic *Yersinia* species have acquired mutations in *Y. pestis* that rendered them non-functional (Chain, P. S., et al., Proc. Natl. Acad. Sci. USA, 101, 13826-13831, (2004), Parkhill, J., et al., Nature, 413, 523-527, (2001)). Some of these encode invasin proteins that function during intestinal invasion in the enteropathogenic *Y. enterocolitica* and *Y. pseudotuberculosis* species, a host niche not colonized by *Y. pestis* (Simonet, M., et al., Infect Immun, 64, 375-379, (1996)). Other genes with lost function in *Y. pestis* include those involved in intermediary metabolism, and these functional losses are theorized to be part of the evolution of *Y. pestis* into an obligate parasitic species with the inability to survive outside the host (Parkhill, J., et al., Nature, 413, 523-527, (2001)). Research on the pathogenesis of *Yersinia* has largely been focused on the 70 kb virulence plasmid that is found in all pathogenic species of *Yersinia.* The sequence of this plasmid, called pYV in *Y. pseudotuberculosis* and pathogenic *Y. enterocolitica* and pCD1 in *Y. pestis,* is remarkably conserved between *Y. pseudotuberculosis* and *Y. pestis* (Chain, P. S., et al., Proc. Natl. Acad. Sci. USA, 101, 13826-13831, (2004)). Accordingly, the more distantly-related *Y. enterocolitica* species harbors a more divergent pYV plasmid, but the virulence gene sequences are highly conserved among all three species (Hu, P., et al., J Bacteriol, 180, 5192-5202, (1998), Snellings, N. J., et al., Infect Immun, 69, 4627-38, (2001)). Focus on this plasmid began when experiments determined that the pYV plasmid is absolutely required for virulence of *Yersinia,* although the plasmid alone cannot restore virulence to specific avirulent strains suggesting that non-pVY genes are also involved in pathogenesis (Heesemann, J., et al., Infect Immun, 46, 105-110, (1984), Heesemann, J. and R. Laufs, J Bacteriol, 155, 761-767, (1983)). A large locus on this plasmid encodes the Ysc-Yop system, a type III secretion system and its associated effector proteins. This system was the first example of a type III secretion apparatus, now identified in many animal and plant microbial pathogens (for review, see Cornelis, G. R., Nat. Rev. Mol. Cell. Biol., 3, 742-752, (2002)). The *Yersinia* Yop-Ysc secretion system includes "injectisome" proteins, translocator effector proteins, and Yop effector proteins. Electron microscopy and labeling studies with various type III secretory systems revealed that the injectisome proteins form a pore spanning the cytoplasmic and outer membranes of the bacteria and project a needle-like structure from the cell surface (Blocker, A., et al., Mol. Microbiol., 39, 652-663, (2001), Kimbrough, T. G. and S. I. Miller, Proc Natl Acad Sci USA, 97, 11008-11013, (2000), Kubori, T., et al., Science, 280, 602-605, (1998), Sukhan, A., et al., J Bacteriol, 183, 1159-1167, (2001)). The translocator proteins appear to interact with host macrophages and polymorphonuclear neutrophils (PMNs), forming a pore-like structure in the host cell membrane (Neyt, C. and G. R. Cornelis, Mol Microbiol, 33, 971-981, (1999)). The assembled secretion apparatus then allows the effector Yops to be translocated across the bacterial cell membranes and injected into the host cell, where they function by interfering with various immune response pathways (Bleves, S. and G. R. Cornelis, Microbes Infect., 2, 1451-1460, (2000), Cornelis, G. R., Nat. Rev. Mol. Cell. Biol., 3, 742-752, (2002)). The *yadA* gene is also present on the pYV plasmid, encoding the YadA adhesin with the ability to bind and adhere to eukaryotic cells (Eitel, J. and P. Dersch, Infect Immun, 70,4880-91, (2002), Skurnik, M., et al., Infect Immun, 62, 1252-61, (1994)). This protein only appears to be functional in the enteropathogenic *Yersinia,* as a frameshift mutation in the *Y. pestis yadA* gene renders it non-functional (Hu, P., et al., J Bacteriol, 180, 5192-5202, (1998)).

The involvement of iron in *Yersinia* infections has long been established. For example, iron-overloaded patients such as those afflicted with β-thalassemia are highly susceptible to *Yersinia* infections (Farmakis, D., et al., Med. Sci. Monit., 9, RA19-22, (2003)). Furthermore, virulence could be restored in specific avirulent *Y*. *pestis* mutants by the addition of heme or heme-containing compounds (Burrows, T. W. and S. Jackson, Br. J. Exp. Pathol., 37, 577-583, (1956)). These early observations with *Yersinia* and other bacteria led researchers to study some of the microbial mechanisms of iron uptake. In mammalian hosts, available iron is extremely limited; intracellular iron is complexed with storage proteins, and extracellular iron is bound by the host proteins transferrin and lactoferrin. These iron-restricted conditions limit the growth of microbial invaders, thus acting as a defense barrier to infection. Many pathogens have evolved the ability to scavenge iron under these iron-poor conditions, effectively "stealing" iron from transferrin or heme-containing compounds. One of the most common mechanisms utilized by bacteria is the synthesis and secretion of siderophores, small molecules with a high affinity for iron (Andrews, S. C., et al., FEMS Microbiol. Rev., 27, 215-237, (2003)). The iron-siderophore complexes are bound by outer membrane receptors on the bacterial cell surface, and through the concerted action of outer membrane, periplasmic, and ABC transporter proteins, iron is transported into the cell. Other outer membrane receptors can directly bind heme and heme-containing compounds, scavenging the iron from these molecules. The role of several *Yersinia* iron uptake systems has been elucidated, while many more putative systems have been identified but not characterized.

Although *Yersinia* can use various siderophores produced by other bacteria and fungi to obtain iron, yersiniabactin is the only *Yersinia-*produced siderophore that has been detected (Baumler, A., et al., Zentralbl. Bakteriol., 278, 416-424, (1993), Rabsch, W. and G. Winkelmann, Biol Met, 4, 244-250, (1991), Reissbrodt, R. and W. Rabsch, Zentralbl Bakteriol Mikrobiol Hyg [A], 268, 306-317, (1988)). The yersiniabactin system is encoded by the *ybt* genes present on the chromosomal high-pathogenicity island (HPI), a locus that is associated with highly pathogenic strains of *Yersinia* (de Almeida, A. M., et al., Microb. Pathog., 14, 9-21, (1993), Rakin, A., et al., J Bacteriol, 177, 2292-2298, (1995)). The *ybt* genes encode proteins involved in the synthesis and secretion of the siderophore yersiniabactin (*ybtS, irp1, irp2, ybtE, ybtT*), as well as the cytoplasmic (*ybtP, ybtQ*) and outer membrane proteins (*psn*/*fyuA*) required for uptake of the iron-yersiniabactin complexes (Carniel, E., Microbes Infect., 3, 561-569, (2001)). Mutations in genes for yersiniabactin synthesis and/or uptake resulted in reduced *Yersinia* virulence in mouse models of infection (Bearden, S. W., et al., Infect. Immun., 65, 1659-1668, (1997), Brem, D., et al., Microbiology, 147, 1115-1127, (2001), Rakin, A., et al., Mol Microbiol, 13, 253-263, (1994)), indicating that this system is an important virulence factor in *Yersinia* pathogenesis. The nucleotide sequence of the *ybt* genes are at least 97% identical between the three pathogenic *Yersinia* species (Carniel, E., Microbes Infect., 3, 561-569, (2001), Chain, P. S., et al., Proc. Natl. Acad. Sci. U S A, 101, 13826-13831, (2004)), and the *Y. pestis* and *Y. pseudotuberculosis ybt* systems were demonstrated to be interchangeable (Perry, R. D., et al., Microbiology, 145 (Pt 5), 1181-1190, (1999)). These analyses indicated that the functions of these homologs are likely conserved among the three species. Furthermore, the HP1 has been discovered in various pathogenic species including some strains of *E. coli, Citrobacter,* and *Klebsiella* (Bach, S., et al., FEMS Microbiol. Lett., 183, 289-294, (2000)). The Ybt proteins expressed by these organisms are quite similar; indeed, antibodies raised against several of the *Yersinia* Ybt proteins recognized the corresponding proteins from the other pathogens (Bach, S., et al., FEMS Microbiol. Lett., 183, 289-294, (2000), Karch, H., et al., Infect Immun, 67, 5994-6001, (1999)). These results suggest that the acquisition of the *ybt* system is relatively recent among these pathogens and may have contributed to the invasive phenotypes associated with many of these serotypes.

Several additional *ybt*-independent iron uptake systems have been detected in *Yersinia* species based on mutation analysis, homology to known iron acquisition proteins, or the presence of iron-responsive regulatory elements. One such regulatory element is the "Fur box," a nucleotide sequence that binds the regulatory protein Fur when it is complexed with iron. The binding of Fe-Fur to a Fur box represses transcription of downstream promoters, and when iron becomes limiting, apo-Fur dissociates from DNA and transcription is derepressed. Fur and its homo logs have been found in most species of bacteria, and regulate many genes in addition to iron uptake systems in diverse organisms (Campoy, S., et al., Microbiology, 148, 1039-1048, (2002), Horsburgh, M. J., et al., Infect Immun, 69, 3744-3754, (2001), Sebastian, S., et al., J Bacteriol, 184, 3965-3974, (2002), Stojiljkovic, I., et al., J Mol Biol, 236, 531-545, (1994)). Analysis of the *Y. pestis* genome identified many genes with Fur boxes upstream of their respective promoters, most of which encoded proteins with homology to known iron uptake systems (Panina, E. M., et al., Nucleic Acids Res, 29, 5195-5206, (2001)). Although few of these genes have been studied for function, several appear to encode iron-siderophore receptor proteins (*omrA, irgA, itrA, ihaB, fauA*) and iron ABC transporters *(itsTUS, itpPTS*)*.* Since *Yersinia* can utilize siderophores produced by other organisms, these proteins may be responsible for the "siderophore piracy" observed with *Yersinia.* Such methods of iron acquisition are common among bacterial pathogens.

Several studies have elucidated the functions of other putative iron uptake systems. For example, the Hmu system of *Y. pestis* was demonstrated to acquire iron through the uptake of heme and heme-containing compounds (Hornung, J. M., et al., Mol Microbiol, 20, 725-39, (1996)). Although the ability to use heme as an iron source seems advantageous for a pathogen, the *Y. pestis hmu* mutant was fully virulent in a mouse model of infection (Thompson, J. M., et al., Infect Immun, 67, 3879-92, (1999)). A second putative heme-uptake system was identified in *Y. pestis* on the basis of sequence homology. The *has* genes of *Y. pestis* are homologs of the hemophore-dependent heme acquisition genes of *Pseudomonas* and *Serratia* (Rossi, M. S., et al., Infect Immun, 69, 6707-6717, (2001)). In these organisms, a hemophore (HasA) is secreted that binds heme and delivers it to bacterial surface receptors (HasR) to transport heme into the cell. The *Y. pestis* HasA protein was determined to be Fur-regulated, secreted, and capable of binding heme. However, a mutation in these genes had no effect on virulence in the mouse, even when a double mutant was tested (Rossi, M. S., et al., Infect Immun, 69, 6707-6717, (2001)). Therefore, the roles of the putative heme uptake systems in pathogenesis remain elusive, and may indicate that heme uptake is more important during infection of non-murine hosts.

The functions of two putative iron ABC transport systems have also been studied in *Yersinia.* The Yfe system can transport iron and manganese in *Y. pestis,* and *yfe* mutants demonstrated reduced virulence in mouse models of infection (Bearden, S. W. and R. D. Perry, Mol. Microbiol., 32, 403-414, (1999)). The second putative iron ABC transporter proteins are encoded by the *yfu* genes, identified by the presence of an upstream Fur box (Gong, S., et al., Infect. Immun., 69, 2829-2837, (2001)). When expressed in E. *coli,* the *yfu* genes restored growth in iron-poor media; however, comparable studies in *Y. pestis* failed to determine a role for Yfu in iron acquisition, and the *yfu-* mutant showed no defect in mouse virulence (Gong, S., et al., Infect. Immun., 69, 2829-2837, (2001)).

### SUMMARY OF THE INVENTION

The present invention is defined by the claims and provides a composition obtainable by a process comprising: providing a culture comprising a *Yersinia enterocolitica* strain ATCC 27729, wherein the *Yersinia enterocolitica* has been grown at 37°C in tryptic soy broth (TSB) medium comprising 160 µM 2,2'-dipyridyl; disrupting the *Yersinia enterocolitica* to result in a mixture comprising disrupted cell membranes; solubilizing the mixture by adding to the mixture a biological detergent to result in a preparation comprising solubilized and unsolubilized proteins; and isolating the unsolubilized polypeptides. The present invention also provides a composition obtainable by a process comprising: providing a culture comprising a *Yersinia pestis* strain KIM6+, wherein the *Yersinia pestis* has been grown at 37°C in TSB medium comprising 160 µM 2,2'-dipyridyl; disrupting the *Yersinia pestis* to result in a mixture comprising disrupted cell membranes; solubilizing the mixture by adding to the mixture a biological detergent to result in a preparation comprising solubilized and unsolubilized proteins; and isolating the unsolubilized polypeptides.

The present disclosure also provides a composition including two isolated polypeptides having molecular weights of 83 kDa , 70 kDa, 66 kDa, or a combination thereof, and two isolated polypeptides having molecular weights of 40 kDa, 38 kDa, or 37 kDa, or a combination thereof, wherein molecular weight is determined by electrophoresis on a sodium dodecyl sulfate-polyacrylamide gel. The polypeptides having a molecular weight of 83 kDa , 70 kDa, or 66 kDa are isolatable from a *Yersinia enterocolitica* when incubated in media containing an iron chelator and not isolatable when grown in the media without the iron chelator. In some disclosures, the composition may include two different 83 kDa polypeptides isolatable from a *Y. enterocolitica* when incubated in media comprising an iron chelator. The composition protects a mouse against challenge with *Y. enterocolitica* ATCC strain 27729. The composition can further include a pharmaceutically acceptable carrier. The polypeptides may be isolatable, or in some aspects isolated from *Y. enterocolitica* is ATCC strain 27729. The composition may further include an isolated polypeptide having a molecular weight of 268 kDa, 92 kDa, 79 kDa, 54 kDa, 45 kDa, 31 kDa, 28 kDa, or a combination thereof, and isolatable from a *Y*. *enterocolitica* when grown in the media without the iron chelator.

The present disclosure also provides a composition including two isolated polypeptides having molecular weights of 83 kDa , 70 kDa, 66 kDa, or a combination thereof, and two isolated polypeptides having molecular weights of 268 kDa, 79 kDa, or 45 kDa, or a combination thereof, wherein molecular weight is determined by electrophoresis on a sodium dodecyl sulfate-polyacrylamide gel. The polypeptides having a molecular weight of 83 kDa , 70 kDa, or 66 kDa are isolatable from a *Yersinia enterocolitica* when incubated in media comprising an iron chelator and not isolatable when grown in the media without the iron chelator. The composition protects a mouse against challenge with *Y. enterocolitica* ATCC strain 27729. The composition can further include a pharmaceutically acceptable carrier. The polypeptides may be isolatable, or in some aspects isolated from *Y. enterocolitica* is ATCC strain 27729.

The present disclosure further provides a composition including isolated polypeptides having molecular weights of 268 kDa, 92 kDa, 83 kDa , 79 kDa, 70 kDa, 66 kDa, 54 kDa, 45 kDa, 40 kDa, 38 kDa, 37 kDa, 31 kDa, and 28 kDa, wherein molecular weight is determined by electrophoresis on a sodium dodecyl sulfate-polyacrylamide gel. The polypeptides are isolatable from a *Yersinia enterocolitica,* and the composition protects a mouse against challenge with *Y. enterocolitica* ATCC strain 27729. The polypeptides may be isolatable, or in some aspects isolated from *Y. enterocolitica* is ATCC strain 27729.

The present disclosure provides a composition including two isolated polypeptides having molecular weights of 94 kDa, 88 kDa, 77 kDa, 73 kDa, or 64 kDa, or a combination thereof, and two isolated polypeptides having molecular weights of 46 kDa, 37 kDa, or a combination thereof, wherein molecular weight is determined by electrophoresis on a sodium dodecyl sulfate-polyacrylamide gel. The polypeptides having a molecular weight of 94 kDa, 88 kDa, 77 kDa, 73 kDa, or 64 kDa are isolatable from a *Yersinia pestis* when incubated in media comprising an iron chelator and not isolatable when grown in the media without the iron chelator. The composition protects a mouse against challenge with *Y. pestis* strain KIM6+. The composition can further include a pharmaceutically acceptable carrier. The polypeptides may be isolatable, or in some aspects isolated from *Y*. *enterocolitica* is ATCC strain 27729. The composition may further include an isolated polypeptide having a molecular weight of 254 kDa, 46 kDa, 37 kDa, 36 kDa, 31 kDa, 28 kDa, or 20 kDa, and isolatable from a *Y. pestis* when grown in the media without the iron chelator. The polypeptides may be isolatable, or in some aspects isolated from *Y. pestis* strain KIM6+.

The present disclosure also provides a composition including two isolated polypeptides having molecular weights of 94 kDa, 88 kDa, 77 kDa, 73 kDa, or 64 kDa, or a combination thereof, and two isolated polypeptides having molecular weights of 254 kDa, 46 kDa, 37 kDa, 36 kDa, 31 kDa, 28 kDa, 20 kDa, or a combination thereof, wherein molecular weight is determined by electrophoresis on a sodium dodecyl sulfate-polyacrylamide gel. The polypeptides having a molecular weight of 94 kDa, 88 kDa, 77 kDa, 73 kDa, or 64 kDa, are isolatable from a *Yersinia pestis* when incubated in media comprising an iron chelator and not isolatable when grown in the media without the iron chelator. The composition protects a mouse against challenge with *Y. pestis* strain KIM6+. The composition can further include a pharmaceutically acceptable carrier. The polypeptides may be isolatable, or in some aspects isolated from *Y. pestis* strain KIM6+.

The present disclosure further provides a composition including isolated polypeptides having molecular weights of 254 kDa, 104 kDa, 99 kDa, 94 kDa , 88 kDa, 77 kDa, 73 kDa, 64 kDa, 60 kDa, 46 kDa, 44 kDa, 37 kDa, 36 kDa, 31 kDa, 28 kDa, and 20 kDa wherein molecular weight is determined by electrophoresis on a sodium dodecyl sulfate-polyacrylamide gel. The polypeptides are isolatable from a *Yersinia pestis,* and the composition protects a mouse against challenge with *Y. pestis* strain KIM6+. The polypeptides may be isolatable, or in some aspects isolated from *Y*. *pestis* strain KIM6+.

The present disclosure provides a method for treating an infection in a subject including administering an effective amount of a composition of the present invention to a subject having or at risk of having an infection caused by a *Yersinia* spp. The subject may be an animal, such as a fish or a mammal, such as a human. The *Yersinia* spp. may be, for example, *Y. enterocolitica* or *Y. pestis,* or *Y. ruckeri.*

The present disclosure also provides a method for treating a symptom in a subject including administering an effective amount of a composition of the present invention to a subject having an infection caused by a *Yersinia* spp. The subject may be an animal, such as a fish or a mammal, such as a human. The *Yersinia* spp. may be, for example, *Y. enterocolitica* or *Y. pestis,* or *Y. ruckeri.* The symptom may be, for example, diarrhea, enteritis, plague, red mouth disease, or a combination thereof.

The present disclosure further provides for treating in infection in a subject including administering an effective amount of a composition to a subject having or at risk of having an infection caused by a *Yersinia* spp., wherein the composition includes antibody that specifically binds a polypeptide of the present disclosure. The antibody may be polyclonal or monoclonal. In one example, the antibody specifically binds two isolated polypeptides having molecular weights of 83 kDa , 70 kDa, 66 kDa, or a combination thereof, wherein the polypeptides are isolatable from a *Yersinia enterocolitica* when incubated in media comprising an iron chelator and not isolatable when grown in the media without the iron chelator. In another example, the antibody specifically binds two isolated polypeptides having molecular weights of 94 kDa, 88 kDa, 77 kDa, 73 kDa, or 64 kDa, or a combination thereof, wherein the polypeptides are isolatable from a *Yersinia pestis* when incubated in media comprising an iron chelator and not isolatable when grown in the media without the iron chelator.

The present disclosure also provides a method for treating a symptom in a subject including administering an effective amount of a composition to a subject having an infection caused by a *Yersinia* spp., wherein the composition includes antibody that specifically binds a polypeptide of the present disclosure. The antibody may be polyclonal or monoclonal. In one example, the antibody specifically binds two isolated polypeptides having molecular weights of 83 kDa , 70 kDa, 66 kDa, or a combination thereof, wherein molecular weight is determined by electrophoresis on a sodium dodecyl sulfate-polyacrylamide gel, wherein the polypeptides are isolatable from a *Yersinia enterocolitica* when incubated in media comprising an iron chelator and not isolatable when grown in the media without the iron chelator. In another example, the antibody specifically binds two isolated polypeptides having molecular weights of 94 kDa, 88 kDa, 77 kDa, 73 kDa, or 64 kDa, or a combination thereof, wherein the polypeptides are isolatable from a *Yersinia pestis* when incubated in media comprising an iron chelator and not isolatable when grown in the media without the iron chelator.

The present disclosure further provides kits for detecting antibody that specifically binds a polypeptide of the present disclosure. The kit includes an isolated polypeptide of the present disclosure, and a reagent that detects an antibody that specifically binds the polypeptide. The polypeptide and the reagent are typically present in separate containers. In one example, the polypeptide may have a molecular weight of 83 kDa , 70 kDa, or 66 kDa, or a combination thereof, wherein the polypeptide is isolatable from a *Yersinia enterocolitica* when incubated in media comprising an iron chelator and not isolatable when grown in the media without the iron chelator. In another example, the polypeptide may have a molecular weight of 94 kDa, 88 kDa, 77 kDa, 73 kDa, or 64 kDa, or a combination thereof, wherein the polypeptide is isolatable from a *Yersinia pestis* when incubated in media comprising an iron chelator and not isolatable when grown in the media without the iron chelator.

### BRIEF DESCRIPTION OF THE FIGURES

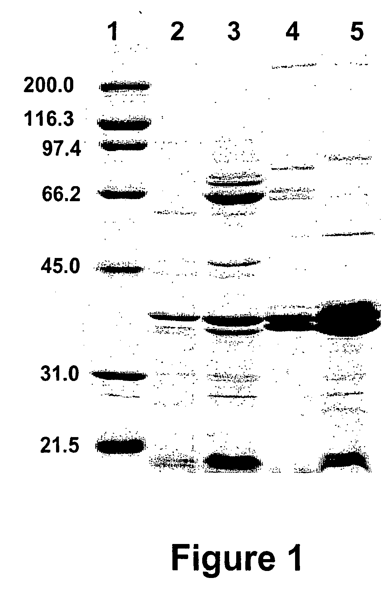

Figure 1. Detergent-insoluble protein-enriched extracts of *Y. enterocolitica* ATCC strain 27729 and *Y. pestis* strain KIM6+ resolved by electrophoresis on a 10% sodium dodecyl sulfate-polyacrylamide gel. The numbers to the left of the gel image denote the molecular weights in kDa of the standards shown in Lane 1. Lane 1, molecular weight standards; Lane 2, *Y. pestis* strain KIM6+ grown in media supplemented with 300 µM FeCl₃; Lane 3, Y. pestis strain KIM6+ grown in media supplemented with 160 µM 2,2-diprydyl; Lane 4, *Y. enterocolitica* ATCC strain 27729 grown in media supplemented with 160 µM 2,2-diprydyl; Lane 5, *Y*. *enterocolitica* ATCC strain 27729 grown in media supplemented with 300 µM FeCl₃.

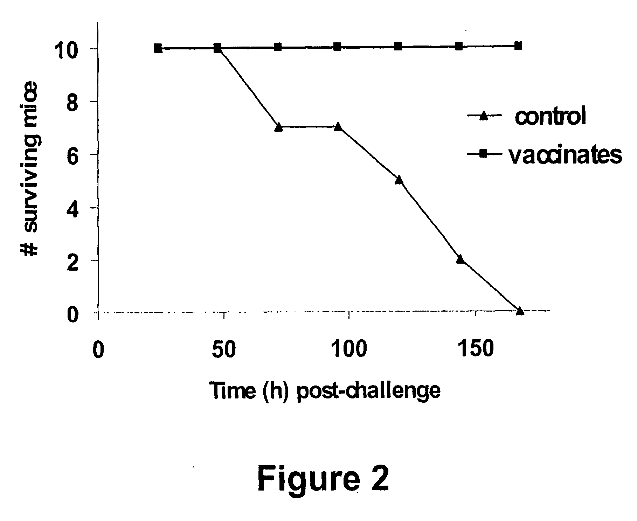

Figure 2. Survival of vaccinated and non-vaccinated mice following challenge with *Y. enterocolitica.* Chart showing survival analysis of mice following immunization with membrane proteins derived from *Y. enterocolitica* strain 27729 grown under iron-limiting conditions and subsequent live challenge with strain 27729. Mortality was recorded for 7 days following challenge.

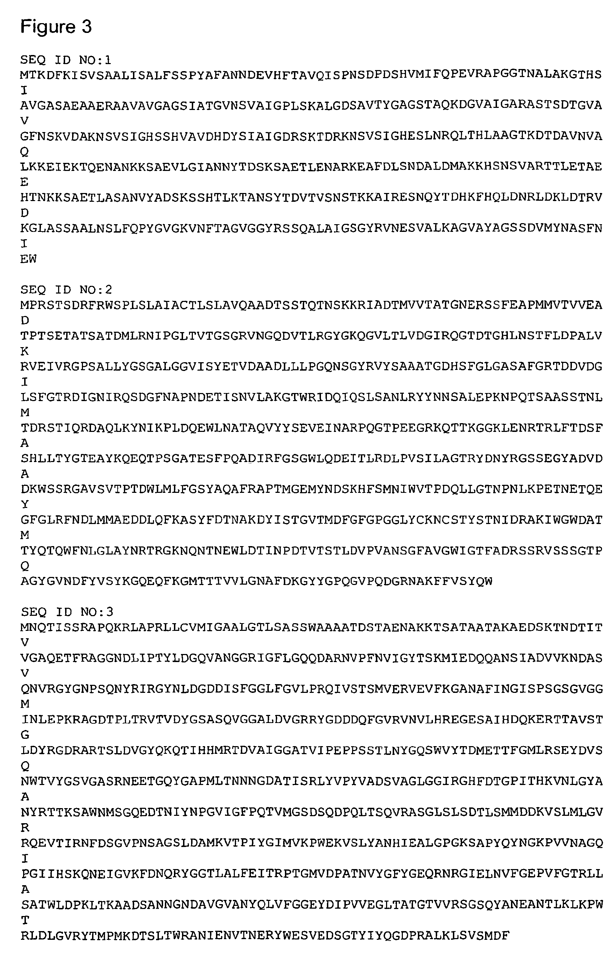

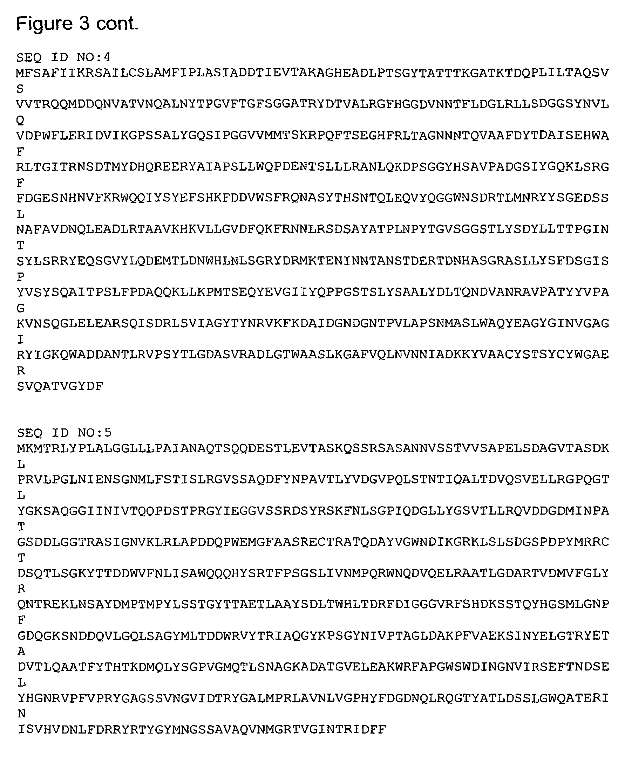

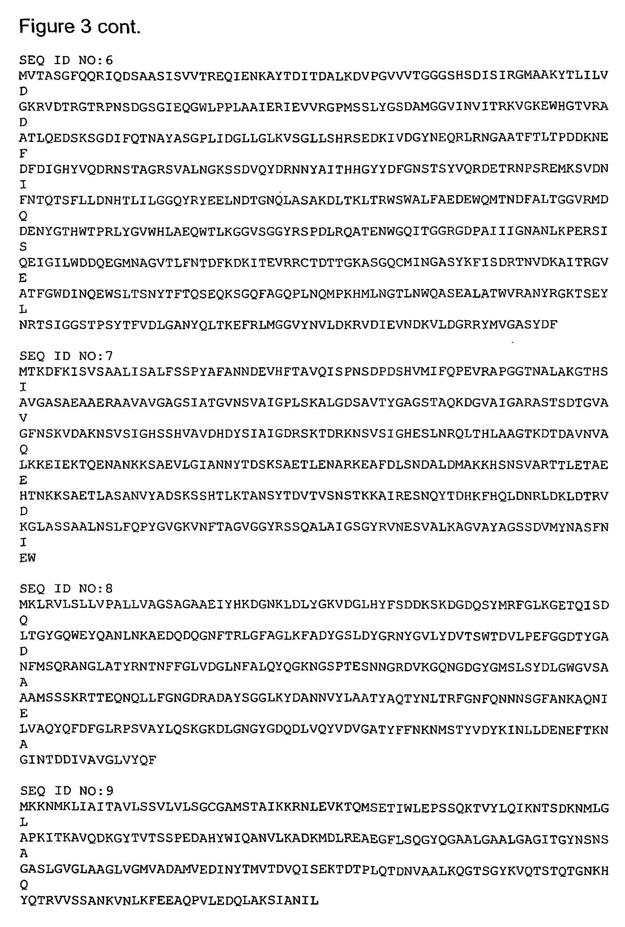

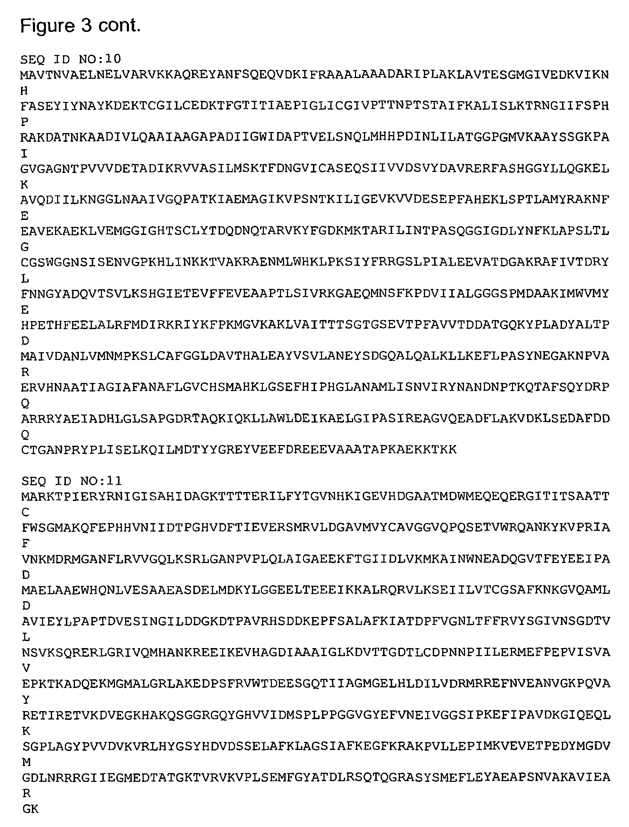

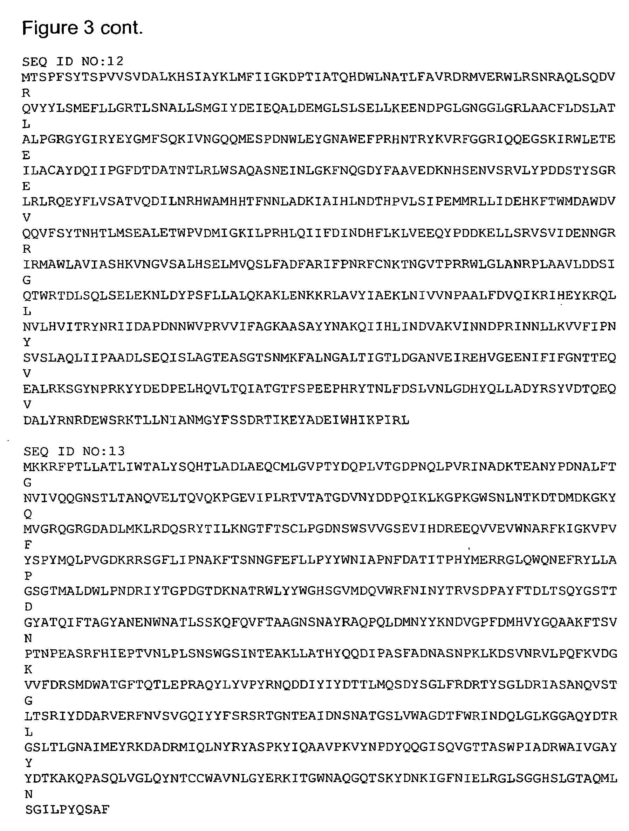

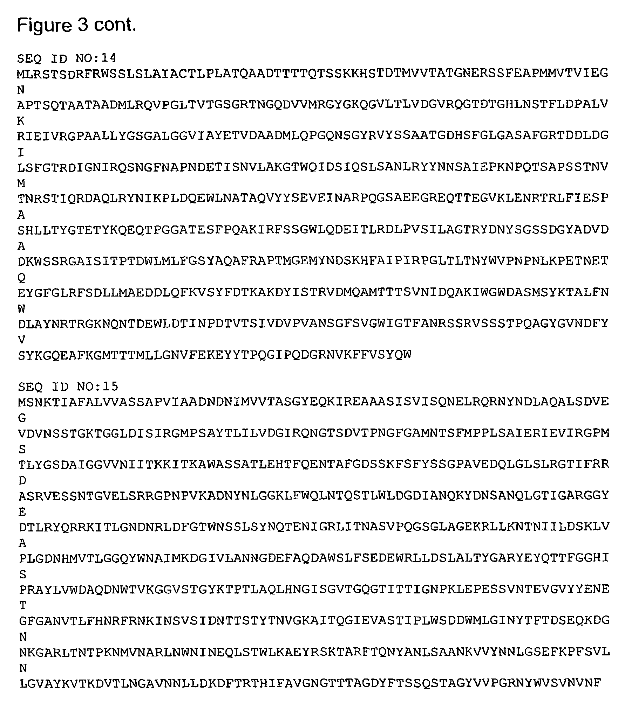

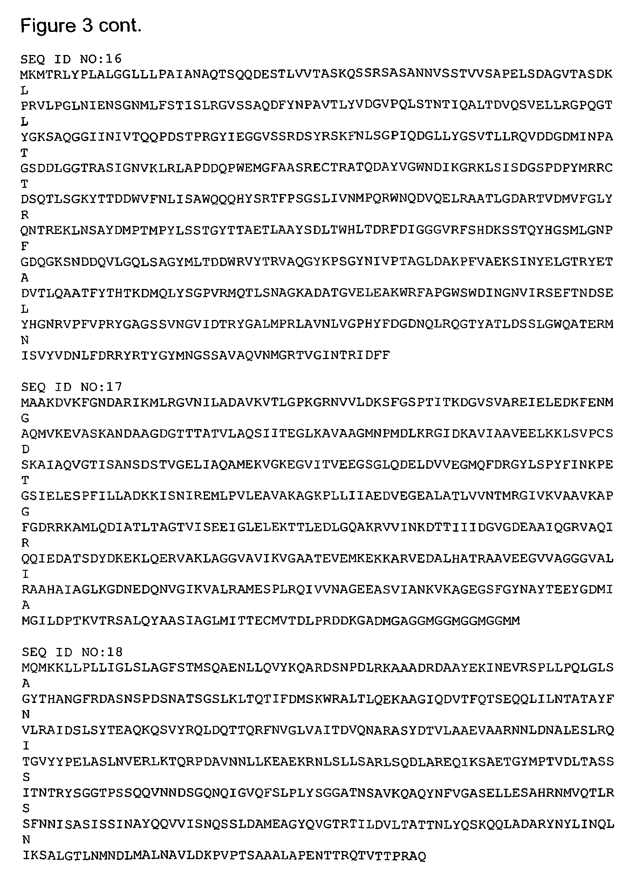

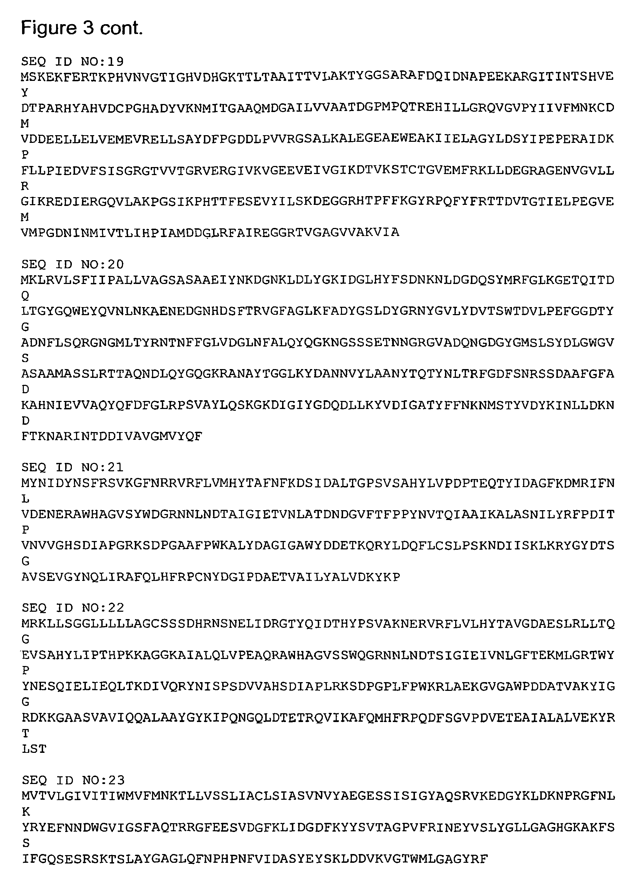

Figure 3. Nucleotide sequences of SEQ ID NOs: 1-23.

### DETAILED DESCRIPTION OF PREFERRED EMBODIMENTS OF THE INVENTION

The present invention provides compositions as defined in the claims.

The present disclosure also provides polypeptides and compositions including polypeptides. As used herein, "polypeptide" refers to a polymer of amino acids linked by peptide bonds. Thus, for example, the terms peptide, oligopeptide, protein, and enzyme are included within the definition of polypeptide. This term also includes post-expression modifications of the polypeptide, for example, glycosylations, acetylations, phosphorylations, and the like. The term polypeptide does not connote a specific length of a polymer of amino acids. A polypeptide may be isolatable directly from a natural source, or can be prepared with the aid of recombinant, enzymatic, or chemical techniques. In the case of a polypeptide that is naturally occurring, such a polypeptide is typically isolated. An "isolated" polypeptide is one that has been removed from its natural environment. For instance, an isolated polypeptide is a polypeptide that has been removed from the cytoplasm or from the outer membrane of a cell, and many of the polypeptides, nucleic acids, and other cellular material of its natural environment are no longer present. An "isolatable" polypeptide is a polypeptide that could be isolated from a particular source. A "purified" polypeptide is one that is at least 60% free, preferably at least 75% free, and most preferably at least 90% free from other components with which they are naturally associated. Polypeptides that are produced outside the organism in which they naturally occur, e.g., through chemical or recombinant means, are considered to be isolated and purified by definition, since they were never present in a natural environment. As used herein, a "polypeptide fragment" refers to a portion of a polypeptide that results from digestion of a polypeptide with a protease. Unless otherwise specified, "a," "an," "the," and "at least one" are used interchangeably and mean one or more than one. The terms "comprises" and variations thereof do not have a limiting meaning where these terms appear in the description and claims.

A polypeptide of the present disclosure may be characterized by molecular weight, mass fingerprint, or the combination thereof. The molecular weight of a polypeptide, typically expressed in kilodaltons (kDa), can be determined using routine methods including, for instance, gel filtration, gel electrophoresis including sodium dodecyl sulfate (SDS) polyacrylamide gel electrophoresis (PAGE), capillary electrophoresis, mass spectrometry, and liquid chromatography including HPLC. Preferably, molecular weight is determined by resolving a polypeptide using an SDS polyacrylamide gel having a stacking gel of about 4% and a resolving gel of about 10% under reducing and denaturing conditions. Unless indicated otherwise, molecular weight refers to molecular weight as determined by SDS-PAGE. As used herein, a "mass fingerprint" refers to a population of polypeptide fragments obtained from a polypeptide after digestion with a protease. Typically, the polypeptide fragments resulting from a digestion are analyzed using a mass spectrometric method. Each polypeptide fragment is characterized by a mass, or by a mass (m) to charge (z) ratio, which is referred to as an "m/z ratio" or an "m/z value". Methods for generating a mass fingerprint of a polypeptide are routine. An example of such a method is disclosed in Example 9.

Polypeptides of the present disclosure may be metal regulated polypeptides. As used herein, a "metal regulated polypeptide" is a polypeptide that is expressed by a microbe at a greater level when the microbe is grown in low metal conditions compared to growth of the same microbe in high metal conditions. Low metal and high metal conditions are described herein. For instance, one class of metal regulated polypeptide produced by *Yersinia* spp. is not expressed at detectable levels during growth of the microbe in high metal conditions but is expressed at detectable levels during growth in low metal conditions. Examples of such metal regulated polypeptides isolatable from *Yersinia enterocolitica* have molecular weights of 83 kDa , 70 kDa, or 66 kDa. In some aspects, *Y. enterocolitica* may produce two different polypeptides each having a molecular weight of 83 kDa and each expressed at detectable levels during growth of the microbe in low metal conditions and not expressed at detectable levels during growth in high metal conditions. Examples of such metal regulated polypeptides isolatable from *Yersinia pestis* have molecular weights of 94 kDa, 88 kDa, 77 kDa, 73 kDa, or 64 kDa.

Another type of metal regulated polypeptide produced by *Yersinia* spp. is expressed at detectable levels during growth of the microbe in high metal conditions but significantly more of the polypeptide is expressed during growth in low metal conditions. The expression of such polypeptides is referred to herein as "enhanced" during growth in low metal conditions. Typically, the expression of a polypeptide during growth in low metal conditions is at least 10% or at least 50% greater than the expression of the polypeptide during growth in high metal conditions. Examples of metal regulated polypeptides showing enhanced expression and isolatable from *Y*. *enterocolitica* have molecular weights of 268 kDa, 79 kDa, or 45 kDa. Examples of metal regulated polypeptides showing enhanced expression and isolatable from *Y*. *pestis* have molecular weights of 254 kDa, 46 kDa, 37 kDa, 36 kDa, 31 kDa, 28 kDa, or 20 kDa. In some aspects, *Y. pestis* may produce two different polypeptides each having a molecular weight of 31 kDa and each showing enhanced expression.

The expression of some polypeptides of the present disclosure is not significantly influenced by the presence of a metal. Examples of such polypeptides isolatable from *Y. enterocolitica* have molecular weights of 92 kDa, 54 kDa, 40 kDa, 38 kDa, 37 kDa, 31 kDa, or 28 kDa. In some aspects, *Y. enterocolitica* may produce two different polypeptides each having a molecular weight of 31 kDa and each not significantly influenced by the presence of a metal. Examples of such polypeptides isolatable from *Y. pestis* have molecular weights of 104 kDa, 99 kDa, 60 kDa, or 44 kDa.

Whether a polypeptide is a metal regulated polypeptide or not can be determined by methods useful for comparing the presence of polypeptides, including, for example, gel filtration, gel electrophoresis including sodium dodecyl sulfate-polyacrylamide gel electrophoresis (SDS-PAGE), capillary electrophoresis, mass spectrometry, and liquid chromatography including HPLC. Separate cultures of a microbe are grown under high metal conditions and under low metal conditions, polypeptides of the present disclosure are isolated as described herein, and the polypeptides present in each culture are resolved and compared. Typically, an equal amount of polypeptides from each culture is used. Preferably, the polypeptides are resolved using an SDS polyacrylamide gel having a stacking gel of about 4% and a resolving gel of about 10% under reducing and denaturing conditions. For instance, 30 micrograms (µg) of total polypeptide from each culture may be used and loaded into wells of a gel. After running the gel and staining the polypeptides with Coomasie Brilliant Blue, the two lanes can be compared. When determining whether a polypeptide is or is not expressed at a detectable level, 30 µg of total polypeptide from a culture is resolved on an SDS-PAGE gel and stained with Coomasie Brilliant Blue using methods known in the art. A polypeptide that can be visualized by eye is considered to be expressed at a detectable level, while a polypeptide that cannot be visualized by eye is considered to be not expressed at a detectable level.

Polypeptides of the present disclosure may have immunogenic activity. "Immunogenic activity" refers to the ability of a polypeptide to elicit an immunological response in an animal. An immunological response to a polypeptide is the development in an animal of a cellular and/or antibody-mediated immune response to the polypeptide. Usually, an immunological response includes but is not limited to one or more of the following effects: the production of antibodies, B cells, helper T cells, suppressor T cells, and/or cytotoxic T cells, directed to an epitope or epitopes of the polypeptide. "Epitope" refers to the site on an antigen to which specific B cells and/or T cells respond so that antibody is produced. The immunogenic activity may be protective. "Protective immunogenic activity" refers to the ability of a polypeptide to elicit an immunological response in an animal that prevents or inhibits infection by *Yersinia* spp., for instance, *Y. enterocolitica* or *Y. pestis.* Whether a polypeptide has protective immunogenic activity can be determined by methods known in the art, for instance as described in example 4 or example 7. For example, a polypeptide of the present disclosure, or combination of polypeptides of the present disclosure, protect a rodent such as a mouse against challenge with a *Yersinia* spp. A polypeptide of the present disclosure may have seroreactive activity. "Seroactive activity" refers to the ability of a candidate polypeptide to react with antibody present in convalescent serum from an animal infected with a *Yersinia* spp., preferably *Y. enterocolitica* or *Y. pestis.* Polypeptides of the present disclosure may have immunoregulatory activity. "Immunoregulatory activity" refers to the ability of a polypeptide to act in a nonspecific manner to enhance an immune response to a particular antigen. Methods for determining whether a polypeptide has immunoregulatory activity are known in the art.

A polypeptide of the present disclosure has the characteristics of a polypeptide expressed by a reference microbe. The characteristics include both molecular weight and mass fingerprint. The reference microbe can be *Y. enterocolitica, Y. pseudotuberculosis, Y. pestis, Y. ruckeri, Y. rohdei, Y. aldovae, Y. bercovieri, Y. frederiksenii, Y. intermedia, Y. kristensenii,* or *Y. moolaretti,* preferably *Y. enterocolitica,* for instance, *Y. enterocolitica* ATCC strain 27729, or *Y. pestis,* for instance, *Y. pestis* strain KIM6+ (Gong et al., Infect. Immun., 69:2829-2837 (2001)).

When the reference microbe is *Y. enterocolitica,* for instance, *Y. enterocolitica* ATCC strain 27729, a candidate polypeptide is considered to be a polypeptide of the present disclosure if it has a molecular weight of 268 kDa, 83 kDa, 79 kDa, 70 kDa, 66 kDa, or 45 kDa, and has a mass fingerprint that is similar to the mass fingerprint of a metal regulated polypeptide expressed by the reference microbe and having a molecular weight of 268 kDa, 83 kDa, 79 kDa, 70 kDa, 66 kDa, or 45 kDa, respectively. Preferably, such polypeptides are metal regulated. For instance, a candidate polypeptide is a polypeptide of the present disclosure if it has a molecular weight of 83 kDa and has a mass fingerprint similar to the mass fingerprint of one of the metal regulated 83 kDa polypeptides produced by the reference strain *Y. enterocolitica* ATCC strain 27729. A candidate polypeptide is also considered to be a polypeptide of the present disclosure if it has a molecular weight of 92 kDa, 54 kDa, 40 kDa, 38 kDa, 37 kDa, 31 kDa, or 28 kDa and has a mass fingerprint that is similar to the mass fingerprint of a polypeptide expressed by the reference microbe and having a molecular weight of 92 kDa, 54 kDa, 40 kDa, 38 kDa, 37 kDa, 31 kDa, or 28 kDa, respectively.

When the reference microbe is *Y. pestis,* for instance, *Y. pestis* strain KIM6+, a candidate polypeptide is considered to be a polypeptide of the present disclosure if it has a molecular weight of 254 kDa, 94 kDa , 88 kDa, 77 kDa, 73 kDa, 64 kDa, 46 kDa, 37 kDa, 36 kDa, 31 kDa, 28 kDa, or 20 kDa, and has a mass fingerprint that is similar to the mass fingerprint of a metal regulated polypeptide expressed by the reference microbe and having a molecular weight of 254 kDa, 94 kDa , 88 kDa, 77 kDa, 73 kDa, 64 kDa, 46 kDa, 37 kDa, 36 kDa, 31 kDa, 28 kDa, or 20 kDa, respectively. Preferably, such polypeptides are metal regulated. For instance, a candidate polypeptide is a polypeptide of the present disclosure if it has a molecular weight of 94 kDa and has a mass fingerprint similar to the mass fingerprint of one of the metal regulated 94 kDa polypeptides produced by the reference strain *Y. pestis* strain KIM6+. A candidate polypeptide is also considered to be a polypeptide of the present disclosure if it has a molecular weight of 104 kDa, 99 kDa, 60 kDa, or 44 kDa and has a mass fingerprint that is similar to the mass fingerprint of a polypeptide expressed by the reference microbe and having a molecular weight of 104 kDa, 99 kDa, 60 kDa, or 44 kDa, respectively.

The polypeptides expressed by a reference microbe and referred to above by molecular weight can be obtained by growth of the reference microbe under low metal conditions and the subsequent isolation of a polypeptide by the processes disclosed herein. A candidate polypeptide is isolatable from a microbe, preferably a gram negative microbe, more preferably, a member of the family Enterobacteriaceae preferably, a member of the genus *Yersinia,* such as *Y. enterocolitica, Y. pseudotuberculosis,* or *Y. pestis.* A candidate polypeptide may also be produced using recombinant, enzymatic, or chemical techniques.

A candidate polypeptide may be evaluated by mass spectrometric analysis to determine whether the candidate polypeptide has a mass fingerprint similar to one of the polypeptides expressed by a reference microbe and referred to above by molecular weight. Typically, the candidate polypeptide is isolated, for instance by resolving the candidate polypeptide by gel electrophoresis and excising the portion of the gel containing the candidate polypeptide. Any gel electrophoresis method that separates polypeptides based on differing characteristics can be used, including 1 dimensional or 2 dimensional gel electrophoresis, as well as liquid chromatographic separation based on, for instance, hydrophobicity, pI, or size. The candidate polypeptide is fragmented, for instance by digestion with a protease. Preferably, the protease cleaves the peptide bond on the carboxy-terminal side of the amino acid lysine and the amino acid arginine, except when the amino acid following the lysine or the arginine is a proline. An example of such a protease is trypsin. Methods for digesting a polypeptide with trypsin are routine and known in the art. An example of such a method is disclosed in Example 9.

Methods for the mass spectrometric analysis of polypeptides are routine and known in the art and include, but are not limited to, matrix assisted laser desorption/ionization time of flight mass spectroscopy (MALDI-TOF MS). Typically, a mixture containing the polypeptide fragments obtained from a candidate polypeptide is mixed with a matrix that functions to transform the laser energy to the sample and produce ionized, preferably monoisotopic, polypeptide fragments. Examples of matrices that can be used include, for instance, sinapinic acid or cyano-4-hydroxycinnamic acid. An example of a method for the analysis of polypeptides by MALDI-TOF MS is described in Example 9. The ionized polypeptide fragments are separated according to their m/z ratio, and detected to yield a spectrum of m/z ratio versus intensity. The spectrum includes m/z values that represent the polypeptide fragments derived from the candidate polypeptide. For any given polypeptide, the amount of each polypeptide fragment resulting from a trypsin digestion should be equimolar. However, it is known that trypsin digestion is not always 100% efficient, for instance, some sites are more efficiently cleaved. Thus, when MALDI-TOF MS is used to determine m/z values, the intensity of each m/z value is typically not identical. Generally, a spectrum has a background level of noise present across most of the x-axis (i.e., the axis having the values of the m/z ratios). This background level of noise varies depending on the running conditions and the machine used, and is easily identified by visual inspection of the spectrum. An m/z value is generally considered to represent a polypeptide fragment when the intensity is at least 2 times greater, 3 times greater, or 4 times greater than the background level of noise. The spectrum usually includes other m/z values that are artifacts resulting from, for instance, incomplete digestion, over digestion, other polypeptides that may be present in the mixture, or the protease used to digest the polypeptide including m/z values resulting from autolysis of the protease. This method of digesting a polypeptide with a protease is recognized by the art as resulting in a mass fingerprint of great specificity that can be used to accurately characterize the polypeptide and distinguish it from other polypeptides.

In this aspect of the disclosure, when a candidate polypeptide is analyzed by mass spectroscopy, preferably both the candidate polypeptide and the polypeptide from the reference microbe are prepared and analyzed together, thereby decreasing any potential artifacts resulting from differences in sample handling and running conditions. Preferably, all reagents used to prepare and analyze the two polypeptides are the same. For instance, the polypeptide from the reference microbe and the candidate polypeptide are isolated under substantially the same conditions, fragmented under substantially the same conditions, and analyzed by MALDI-TOF MS on the same machine under substantially the same conditions. A mass fingerprint of a candidate polypeptide is considered to be similar to the mass fingerprint of a polypeptide from a reference microbe when at least 80%, at least 90%, at least 95%, or substantially all of the m/z values present in the spectrum of the reference microbe polypeptide and above the background level of noise are also present in the spectrum of the candidate polypeptide.

In another aspect, a polypeptide is considered to be a polypeptide of the present disclosure if it has a molecular weight of a reference polypeptide described in Table 1 or Table 2 and has a mass fingerprint that includes the population of polypeptide fragments of the reference polypeptide as listed in Table 1 or Table 2. For instance, a polypeptide of the present disclosure includes a polypeptide of 83 kDa and a mass fingerprint that includes polypeptide fragments having masses of 686.37, 975.45, 1000.53, 1015.46, 1140.65, 1169.68, 1170.64, 1197.57, 1342.55, 1356.74, 1394.67, 1452.73, 1476.72, 1520.76, 1692.77, 1715.75, 1828.79, 1960.91, 2013.02, 2018.95, 2040.97, 2163.05, 2225.03, 2416.19, and 3174.44, or a mass fingerprint that includes polypeptide fragments having masses of 1001.49, 1103.57, 1139.57, 1154.51, 1170.49, 1208.59, 1213.67, 1337.70, 1452.86, 1567.84, 1633.85, 1650.82, 1659.91, 1708.77, 1748.95, 1849.92, 1986.98, 2103.95, 2111.03, 2163.11, 2386.19, 2452.09, 2537.34, and 3422.66. The mass fingerprint of a candidate polypeptide can be determined by a mass spectrometric method, for instance by MALDI-TOF MS. The mass fingerprint of a candidate polypeptide will generally have additional polypeptide fragments and therefore additional m/z values other than those listed for a polypeptide in Table 1 or Table 2. Preferably, when the candidate polypeptide is being compared to a polypeptide in Table 1 or Table 2, the candidate polypeptide is obtained from a *Y. pestis, Y. pseudotuberculosis,* or *Y. enterocolitica,* more preferably, *Y. enterocolitica* or *Y. pestis.* A candidate polypeptide can be obtained by growth of a microbe under low metal conditions and the subsequent isolation of a polypeptide by the processes described herein.

It is well known in the art that modifications of amino acids can be accidentally introduced during sample handling, such as oxidation, and formation of carbamidomethyl derivatives. Further, these types of modifications alter the m/z value of a polypeptide fragment. For instance, if a polypeptide fragment contains a methoinine that is oxidized the m/z value will be increased by 16 relative to the same fragment that does not contain the oxidized methionine. Accordingly, those polypeptide fragments in Tables 1 and 2 having the notation "oxidation (M)" have an m/z value that is increased by 16 relative to the same fragment that does not contain the oxidized methionine. It is understood that the polypeptide fragments of Table 1 and Table 2 can be modified during sample handling.

**Table 1. Characteristics of polypeptides obtained from Y. enterocolitica.**

| polypeptide designation | approximate molecular weight in kilodaltons (kDa)¹ | mass of polypeptide fragments resulting from trypsin digest² | predicted amino acid sequence of the polypeptide fragment (SEQ ID Numbers listed in parenthesis) |
|---|---|---|---|
| Lw545 | 268 | 928.45 | FHQLDNR (SEQ ID No: 24) |
| | | 1139.57 | VNFTAGVGGYR (SEQ ID No: 25) |
| | | 1311.65 | NSVSIGHESLNR (SEQ ID No: 26) |
| | | 1439.69 | ASTSDTGVAVGFNSK (SEQ ID No: 27) |
| | | 1525.73 | SAETLASANVYADSK (SEQ ID No:28) |
| | | 1554.69 | EAFDLSNDALDMAK + Oxidation (M) (SEQ ID No: 29) |
| | | 1580.77 | SAEVLGIANNYTDSK (SEQ ID No: 30) |
| | | 1595.78 | ALGDSAVTYGAGSTAQK (SEQ ID No:31) |
| | | 1682.78 | EAFDLSNDALDMAKK + Oxidation (M)(SEQ ID No:32) |
| | | 2109.18 | AAVAVGAGSIATGVNSVAIGPLSK (SEQ ID No:33) |
| Lw391A | 83 | 686.37 | DIGNIR (SEQ ID No:34) |
| | | 975.45 | FFVSYQW (SEQ ID No:35) |
| | | 1000.53 | VNGQDVTLR (SEQ ID No:36) |
| | | 1015.46 | ASYFDTNAK (SEQ ID No:37) |
| | | 1140.65 | DLPVSILAGTR (SEQ ID No:38) |
| | | 1169.68 | QGVLTLVDGIR (SEQ ID No:39) |
| | | 1170.64 | NIPGLTVTGSGR (SEQ ID No:40) |
| | | 1197.57 | YYNNSALEPK (SEQ ID No:41) |
| | | 1342.55 | APTMGEMYNDSK (SEQ ID No:42) |
| | | 1356.74 | IDQIQSLSANLR (SEQ ID No:43) |
| | | 1394.67 | TDDVDGILSFGTR (SEQ ID No:44) |
| | | 1452.73 | GMTTTVVLGNAFDK (SEQ ID No:45) |
| | | 1476.72 | IADTMVVTATGNER (SEQ ID No:46) |
| | | 1520.76 | FGSGWLQDEITLR (SEQ ID No:47) |
| | | 1692.77 | NPQTSAASSTNLMTDR (SEQ ID No:48) |
| | | 1715.75 | FNDLMMAEDDLQFK (SEQ ID No:49) |
| | | 1828.79 | GSSEGYADVDADKWSSR (SEQ ID No:50) |
| | | 1960.91 | QEQTPSGATESFPQADIR (SEQ ID No:51) |
| | | 2013.02 | QGTDTGHLNSTFLDPALVK (SEQ ID No:52) |
| | | 2018.95 | QSDGFNAPNDETISNVLAK (SEQ ID No:53) |
| | | 2040.97 | VYSAAATGDHSFGLGASAFGR (SEQ ID No:54) |
| | | 2163.05 | LFTDSFASHLLTYGTEAYK (SEQ ID No:55) |
| | | 2225.03 | VSSSGTPQAGYGVNDFYVSYK (SEQ ID No:56) |
| | | 2416.19 | GAVSVTPTDWLMLFGSYAQAFR (SEQ ID No:57) |
| | | 3174.44 | SSFEAPMMVTWEADTPTSETATSATDMLR (SEQ ID No:58) |
| Lw391B | 83 | 1001.49 | NDASVQNVR (SEQ ID No:59) |
| | | 1103.57 | IGFLGQQDAR (SEQ ID No:60) |
| | | 1139.57 | VNLGYAANYR (SEQ ID No:61) |
| | | 1154.51 | GYGNPSQNYR (SEQ ID No:62) |
| | | 1170.49 | YGDDDQFGVR (SEQ ID No:63) |
| | | 1208.59 | GHFDTGPITHK (SEQ ID No:64) |
| | | 1213.67 | LLASATWLDPK (SEQ ID No:65) |
| | | 1337.70 | NVPFNVIGYTSK (SEQ ID No:66) |
| | | 1452.86 | LKPWTRLDLGVR (SEQ ID No:67) |
| | | 1567.84 | VSLYANHIEALGPGK (SEQ ID No:68) |
| | | 1633.85 | GIELNVFGEPVFGTR (SEQ ID No:69) |
| | | 1650.82 | TNDTITVVGAQETFR (SEQ ID No:70) |
| | | 1659.91 | VTPIYGIMVKPWEK (SEQ ID No:71) |
| | | 1708.77 | NFDSGVPNSAGSLDAMK (SEQ ID No:72) |
| | | 1748.95 | LYVPYVADSVAGLGGIR (SEQ ID No:73) |
| | | 1849.92 | VTVDYGSASQVGGALDVGR (SEQ ID No:74) |
| | | 1986.98 | AGGNDLIPTYLDGQVANGGR (SEQ ID No:75) |
| | | 2103.95 | SEYDVSQNWTVYGSVGASR (SEQ ID No:76) |
| | | 2111.03 | GYNLDGDDISFGGLFGVLPR (SEQ ID No:77) |
| | | 2163.11 | SGSQYANEANTLKLKPWTR (SEQ ID No:78) |
| | | 2386.19 | GANAFINGISPSGSGVGGMINLEPK (SEQ ID No:79) |
| | | 2452.09 | NEETGQYGAPMLTNNNGDATISR (SEQ ID No:80) |
| | | 2537.34 | SAPYQYNGKPVVNAGQIPGIIHSK (SEQ ID No:81) |
| | | 3422.66 | YGGTLALFEITRPTGMVDPATNVYGFYGEQR (SEQ ID No:82) |
| Lw392 | 79 | 836.44 | YDTVALR (SEQ ID No:83) |
| | | 1017.59 | VLLGVDFQK (SEQ ID No:84) |
| | | 1070.48 | FDDVWSFR (SEQ ID No:85) |
| | | 1085.50 | SVQATVGYDF (SEQ ID No:86) |
| | | 1131.59 | ADLGTWAASLK (SEQ ID No:87) |
| | | 1188.55 | QWADDANTLR (SEQ ID No:88) |
| | | 1214.63 | VNSQGLELEAR (SEQ ID No:89) |
| | | 1235.65 | AVPATYYVPAGK (SEQ ID No:90) |
| | | 1255.66 | LSVIAGYTYNR (SEQ ID No:91) |
| | | 1263.65 | VPSYTLGDASVR (SEQ ID No:92) |
| | | 1360.66 | RPQFTSEGHFR (SEQ ID No:93) |
| | | 1496.67 | GFFDGESNHNVFK (SEQ ID No:94) |
| | | 1501.79 | GAFVQLNVNNIADK (SEQ ID No:95) |
| | | 1614.75 | WQQIYSYEFSHK (SEQ ID No:96) |
| | | 1652.77 | GFFDGESNHNVFKR (SEQ ID No:97) |
| | | 1717.82 | GFHGGDVNNTFLDGLR (SEQ ID No:98) |
| | | 1770.85 | RWQQIYSYEFSHK (SEQ ID No:99) |
| | | 1819.86 | AGHEADLPTSGYTATTTK (SEQ ID No:100) |
| | | 1827.01 | TDQPLILTAQSVSVVTR (SEQ ID No:101) |
| | | 2004.92 | DPSGGYHSAVPADGSIYGQK (SEQ ID No:102) |
| | | 2066.02 | GPSSALYGQSIPGGVVMMTSK (EQ ID No:103) |
| | | 2119.91 | KYVAACYSTSYCYWGAER (SEQ ID No:104) |
| | | 2299.22 | YAIAPSLLWQPDENTSLLLR (SEQ ID No:105) |
| | | 2307.15 | LLSDGGSYNVLQVDPWFLER (SEQ ID No:106) |
| | | 2782.23 | QNASYTHSNTQLEQVYQGGWNSDR (SEQ ID No:107) |
| | | 2911.35 | LTAGNNNTQVAAFDYTDAISEHWAFR (SEQ ID No:108) |
| | | 3023.42 | RYEQSGVYLQDEMTLDNWHLNLSGR (SEQ ID No:109) |
| | | 3286.53 | QQMDDQNVATVNQALNYTPGVFTGFSGGATR (SEQ ID No:110) |
| Lw393 | 70 | 713.42 | VPFVPR (SEQ ID No:111) |
| | | 759.42 | TVGINTR (SEQ ID No:112) |
| | | 806.41 | YGALMPR (SEQ ID No:113) |
| | | 819.42 | FDIGGGVR (SEQ ID No:114) |
| | | 919.48 | GPQGTLYGK (SEQ ID No:115) |
| | | 1023.50 | GYIEGGVSSR (SEQ ID No:116) |
| | | 1051.53 | SINYELGTR (SEQ ID No:117) |
| | | 1186.57 | WNQDVQELR (SEQ ID No:118) |
| | | 1199.60 | TVDMVFGLYR (SEQ ID No:119) |
| | | 1394.68 | YGAGSSVNGVIDTR (SEQ ID No:120) |
| | | 1436.66 | LSLSDGSPDPYMR (SEQ ID No:121) |
| | | 1479.70 | ATQDAYVGWNDIK (SEQ ID No:122) |
| | | 1540.80 | INISVHVDNLFDR (SEQ ID No:123) |
| | | 1545.80 | TFPSGSLIVNMPQR (SEQ ID No:124) |
| | | 1564.76 | KLSLSDGSPDPYMR (SEQ ID No:125) |
| | | 1667.72 | SEFTNDSELYHGNR (SEQ ID No:126) |
| | | 1730.85 | FAPGWSWDINGNVIR (SEQ ID No:127) |
| | | 1789.81 | LAPDDQPWEMGFAASR (SEQ ID No:128) |
| | | 1904.85 | TYGYMNGSSAVAQVNMGR (SEQ ID No:129) |
| | | 1981.02 | SAQGGIINIVTQQPDSTPR (SEQ ID No:130) |
| | | 1982.93 | QGTYATLDSSLGWQATER (SEQ ID No:131) |
| | | 1995.94 | DMQLYSGPVGMQTLSNAGK (SEQ ID No:132) |
| | | 2009.89 | SSTQYHGSMLGNPFGDQGK (SEQ ID No:133) |
| | | 2027.02 | LAVNLVGPHYFDGDNQLR (SEQ ID No:134) |
| | | 2058.99 | LRLAPDDQPWEMGFAASR (SEQ ID No:135) |
| | | 2132.93 | QVDDGDMINPATGSDDLGGTR (SEQ ID No:136) |
| | | 2162.17 | FNLSGPIQDGLLYGSVTLLR (SEQ ID No:137) |
| | | 2274.20 | VLPGLNIENSGNMLFSTISLR (SEQ ID No:138) |
| | | 2363.13 | SEFTNDSELYHGNRVPFVPR (SEQ ID No:139) |
| | | 2377.30 | SKFNLSGPIQDGLLYGSVTLLR (SEQ ID No:140) |
| | | 2383.07 | SNDDQVLGQLSAGYMLTDDWR (SEQ ID No:141) |
| | | 2563.22 | SASANNVSSTWSAPELSDAGVTASDK (SEQ ID No:142) |
| | | 2657.23 | YTTDDWVFNLISAWQQQHYSR (SEQ ID No:143) |
| | | 2833.50 | IAQGYKPSGYNIVPTAGLDAKPFVAEK (SEQ ID No:144) |
| | | 2929.46 | SASANNVSSTWSAPELSDAGVTASDKLPR (SEQ ID No:145) |
| Lw550 | 66 | 867.49 | VSGLLSHR (SEQ ID No:146) |
| | | 881.42 | TSEYLNR (SEQ ID No:147) |
| | | 883.43 | EWHGTVR (SEQ ID No:148) |
| | | 1020.59 | YTLILVDGK (SEQ ID No:149) |
| | | 1086.57 | RVDIEVNDK (SEQ ID No:150) |
| | | 1167.61 | VGKEWHGTVR (SEQ ID No:151) |
| | | 1176.69 | YTLILVDGKR (SEQ ID No:152) |
| | | 1207.63 | LMGGVYNVLDK (SEQ ID No:153) |
| | | 1345.72 | IQDSAASISVVTR (SEQ ID No:154) |
| | | 1748.72 | MDQDENYGTHWTPR (SEQ ID No:155) |
| | | 1753.77 | NEFDFDIGHYVQDR (SEQ ID No:156) |
| | | 1850.95 | DVPGVVVTGGGSHSDISIR (SEQ ID No:157) |
| | | 2520.27 | GTRPNSDGSGIEQGWLPPLAAIER (SEQ ID No:158) |
| | | 2606.16 | NNYAITHHGYYDFGNSTSYVQR (SEQ ID No:159) |
| | | 2942.50 | AYTDITDALKDVPGVVVTGGGSHSDISIR (SEQ ID No:160) |
| | | 3085.41 | NGAATFTLTPDDKNEFDFDIGHYVQDR (SEQ ID No:161) |
| Lw552 | 45 | 1139.57 | VNFTAGVGGYR (SEQ ID No:162) |
| | | 1208.61 | SSQALAIGSGYR (SEQ ID No:163) |
| | | 1311.65 | NSVSIGHESLNR (SEQ ID No:164) |
| | | 1439.69 | ASTSDTGVAVGFNSK (SEQ ID No:165) |
| | | 1500.74 | TTLETAEEHTNKK (SEQ ID No:166) |
| | | 1525.73 | SAETLASANVYADSK (SEQ ID No:167) |
| | | 1580.77 | SAEVLGIANNYTDSK (SEQ ID No:168) |
| | | 1595.78 | ALGDSAVTYGAGSTAQK (SEQ ID No:169) |
| Lw555 | 37 | 704.42 | LGFAGLK (SEQ ID No:170) |
| | | 880.43 | ADAYSGGLK (SEQ ID No:171) |
| | | 970.38 | DGDQSYMR (SEQ ID No:172) |
| | | 1121.57 | DGNKLDLYGK (SEQ ID No:173) |
| | | 1279.54 | AEDQDQGNFTR (SEQ ID No:174) |
| | | 1294.58 | VDGLHYFSDDK (SEQ ID No:175) |
| | | 1334.67 | INLLDENEFTK (SEQ ID No:176) |
| | | 1509.71 | VDGLHYFSDDKSK (SEQ ID No:177) |
| | | 1907.96 | NAGINTDDIVAVGLVYQF (SEQ ID No:178) |
| | | 2245.12 | NTNFFGLVDGLNFALQYQGK (SEQ ID No:179) |
| | | 2324.11 | YDANNVYLAATYAQTYNLTR (SEQ ID No:180) |
| | | 2642.22 | GETQISDQLTGYGQWEYQANLNK (SEQ ID No:181) |
| | | 2984.54 | AQNIELVAQYQFDFGLRPSVAYLQSK (SEQ ID No:182) |
| | | 3087.49 | FGLKGETQISDQLTGYGQWEYQANLNK (SEQ ID No:183) |
| Lw557 | 31 | 863.51 | TVYLQIK (SEQ ID No:184) |
| | | 1403.71 | NTSDKNMLGLAPK + Oxidation (M) (SEQ ID No:185) |
| | | 1615.81 | FEEAQPVLEDQLAK (SEQ ID No:186) |
| | | 1779.83 | TQMSETIWLEPSSQK + Oxidation (M) (SEQ ID No:187) |
| | | 1875.92 | VQTSTQTGNKHQYQTR (SEQ ID No:188) |
| | | 2070.10 | VNLKFEEAQPVLEDQLAK (SEQ ID No:189) |
| | | 2378.15 | GYTVTSSPEDAHYWIQANVLK (SEQ ID No:190) |

| | | | |
|---|---|---|---|
| 1. Molecular weight as determined by SDS-PAGE. 2. The mass of a polypeptide fragment can be converted to m/z value by adding 1 to the mass. Each mass includes a range of plus or minus 1 Da or plus or minus 300 ppm. | | | |

**Table 2. Characteristics of polypeptides obtained from Y. pestis.**

| polypeptide designation | approximate molecular weight in kilodaltons (kDa)¹ | mass of polypeptide fragments resulting from trypsin digest² | predicted amino acid sequence of the polypeptide fragment |
|---|---|---|---|
| Lw529 | 104 | 643.43 | ALISLK (SEQ ID No:191) |
| | | 684.36 | SIYFR (SEQ ID No:192) |
| | | 770.49 | ILIGEVK (SEQ ID No:193) |
| | | 840.46 | NPVARER (SEQ ID No:194) |
| | | 898.55 | AVQDIILK (SEQ ID No:195) |
| | | 961.55 | YPLISELK (SEQ ID No:196) |
| | | 1136.61 | NGIIFSPHPR (SEQ ID No:197) |
| | | 1276.63 | EAGVQEADFLAK (SEQ ID No:198) |
| | | 1292.62 | NFEEAVEKAEK (SEQ ID No:199) |
| | | 1385.65 | VVDESEPFAHEK (SEQ ID No:200) |
| | | 1409.76 | NGGLNAAIVGQPATK (SEQ ID No:201) |
| | | 1421.84 | AAALAAADARIPLAK (SEQ ID No:202) |
| | | 1497.82 | AVTNVAELNELVAR (SEQ ID No:203) |
| | | 1566.75 | QTAFSQYDRPQAR (SEQ ID No:204) |
| | | 1678.89 | LLKEFLPASYNEGAK (SEQ ID No:205) |
| | | 1683.82 | YAEIADHLGLSAPGDR (SEQ ID No:206) |
| | | 1725.93 | GSLPIALEEVATDGAKR (SEQ ID No:207) |
| | | 1872.90 | EYANFSQEQVDKIFR (SEQ ID No:208) |
| | | 1990.91 | NHFASEYIYNAYKDEK (SEQ ID No:209) |
| | | 2020.06 | ILINTPASQGGIGDLYNFK (SEQ ID No:210) |
| | | 2182.01 | EYVEEFDREEEVAAATAPK (SEQ ID No:211) |
| | | 2584.21 | YNANDNPTKQTAFSQYDRPQAR (SEQ ID No:212) |
| | | 2842.48 | AAYSSGKPAIGVGAGNTPVVVDETADIKR (SEQ ID No:213) |
| Lw530 | 99 | 1190.64 | ILFYTGVNHK (SEQ ID No:214) |
| | | 1513.80 | YRNIGISAHIDAGK (SEQ ID No:215) |
| | | 1590.77 | HSDDKEPFSALAFK (SEQ ID No:216) |
| | | 1596.83 | IATDPFVGNLTFFR (SEQ ID No:217) |
| | | 1636.82 | YLGGEELTEEEIKK (SEQ ID No:218) |
| | | 1670.86 | MEFPEPVISVAVEPK (SEQ ID No:219) |
| | | 1713.93 | EFIPAVDKGIQEQLK (SEQ ID No:220) |
| | | 1718.96 | LGANPVPLQLAIGAEEK (SEQ ID No:221) |
| | | 1750.91 | VYSGIVNSGDTVLNSVK (SEQ ID No:222) |
| | | 1819.92 | EFNVEANVGKPQVAYR (SEQ ID No:223) |
| | | 1863.01 | EEIKEVHAGDIAAAIGLK (SEQ ID No:224) |
| | | 1966.91 | LHYGSYHDVDSSELAFK (SEQ ID No:225) |
| | | 2122.10 | VYSGIVNSGDTVLNSVKSQR (SEQ ID No:226) |
| Lw531 | 94 | 961.44 | NRDEWSR (SEQ ID No:227) |
| | | 1167.49 | YEYGMFSQK + Oxidation (M) (SEQ ID No:228) |
| | | 1257.64 | VSVIDENNGRR (SEQ ID No:229) |
| | | 1371.63 | VLYPDDSTYSGR (SEQ ID No:230) |
| | | 1383.64 | EENDPGLGNGGLGR (SEQ ID No:231) |
| | | 1408.71 | IIDAPDNNWVPR (SEQ ID No:232) |
| | | 1520.82 | NLDYPSFLLALQK (SEQ ID No:233) |
| | | 1668.86 | EYADEIWHIKPIR (SEQ ID No:234) |
| | | 1685.79 | SYVDTQEQVDALYR (SEQ ID No:235) |
| | | 1713.78 | GYGIRYEYGMFSQK + Oxidation (M) (SEQ ID No:236) |
| | | 1716.81 | TLLNIANMGYFSSDR + Oxidation (M) (SEQ ID No:237) |
| | | 1796.92 | TSPFSYTSPVVSVDALK (SEQ ID No:238) |
| | | 1832.92 | LVEEQYPDDKELLSR (SEQ ID No:239) |
| | | 1844.91 | KTLLNIANMGYFSSDR + Oxidation (M) (SEQ ID No:240) |
| | | 2218.12 | IAIHLNDTHPVLSIPEMMR + 2 Oxidation (M) (SEQ ID No:241) |
| | | 2426.09 | FNQGDYFAAVEDKNHSENVSR (SEQ ID No:242) |
| Lw532 | 88 | 888.51 | YIQAAVPK (SEQ ID No:243) |
| | | 926.46 | FNINYTR (SEQ ID No:244) |
| | | 945.53 | SGFLIPNAK (SEQ ID No:245) |
| | | 960.54 | IGFNIELR (SEQ ID No:246) |
| | | 1171.60 | AQYLYVPYR (SEQ ID No:247) |
| | | 1176.57 | GLQWQNEFR (SEQ ID No:248) |
| | | 1289.64 | ITGWNAQGQTSK (SEQ ID No:249) |
| | | 1332.67 | RGLQWQNEFR (SEQ ID No:250) |
| | | 1357.66 | EEQVVEVWNAR (SEQ ID No:251) |
| | | 1403.74 | IASANQVSTGLTSR (SEQ ID No:252) |
| | | 1418.68 | FTSVNPTNPEASR (SEQ ID No:253) |
| | | 1507.73 | IYTGPDGTDKNATR (SEQ ID No:254) |
| | | 1578.78 | FNVSVGQIYYFSR (SEQ ID No:255) |
| | | 1672.80 | QFQVFTAAGNSNAYR (SEQ ID No:256) |
| | | 1735.83 | TVTATGDVNYDDPQIK (SEQ ID No:257) |
| | | 2400.17 | LLATHYQQDIPASFADNASNPK (SEQ ID No:258) |
| | | 2665.28 | VYNPDYQQGISQVGTTASWPIADR (SEQ ID No:259) |
| Lw533 | 77 | 686.37 | DIGNIR (SEQ ID No:260) |
| | | 784.49 | RIEIVR (SEQ ID No:261) |
| | | 858.41 | VSYFDTK (SEQ ID No:262) |
| | | 952.50 | AKDYISTR (SEQ ID No:263) |
| | | 1140.65 | DLPVSILAGTR (SEQ ID No:264) |
| | | 1155.66 | QGVLTLVDGVR (SEQ ID No:265) |
| | | 1170.64 | QVPGLTVTGSGR (SEQ ID No:266) |
| | | 1197.57 | YYNNSAIEPK (SEQ ID No:267) |
| | | 1402.71 | EQTTEGVKLENR (SEQ ID No:268) |
| | | 1408.68 | TDDLDGILSFGTR (SEQ ID No:269) |
| | | 1482.73 | TALFNWDLAYNR (SEQ ID No:270) |
| | | 1522.71 | EYYTPQGIPQDGR (SEQ ID No:271) |
| | | 1550.77 | FSSGWLQDEITLR (SEQ ID No:272) |
| | | 1617.74 | HSTDTMVVTATGNER (SEQ ID No:273) |
| | | 1674.78 | QEQTPGGATESFPQAK (SEQ ID No:274) |
| | | 1745.84 | KHSTDTMVVTATGNER (SEQ ID No:275) |
| | | 1787.92 | GTWQIDSIQSLSANLR (SEQ ID No:276) |
| | | 1819.96 | IRFSSGWLQDEITLR (SEQ ID No:277) |
| | | 1851.87 | VDMQAMTTTSVNIDQAK (SEQ ID No:278) |
| | | 1940.75 | YDNYSGSSDGYADVDADK (SEQ ID No:279) |
| | | 2013.02 | QGTDTGHLNSTFLDPALVK (SEQ ID No:280) |
| | | 2017.97 | QSNGFNAPNDETISNVLAK (SEQ ID No:281) |
| | | 2056.96 | VYSSAATGDHSFGLGASAFGR (SEQ ID No:282) |
| | | 2168.01 | VSSSTPQAGYGVNDFYVSYK (SEQ ID No:283) |
| | | 2169.10 | LFIESPASHLLTYGTETYK (SEQ ID No:284) |
| | | 2426.25 | TRLFIESPASHLLTYGTETYK (SEQ ID No:285) |
| | | 2457.00 | YDNYSGSSDGYADVDADKWSSR (SEQ ID No:286) |
| | | 2828.33 | VSSSTPQAGYGVNDFYVSYKGQEAFK (SEQ ID No:287) |
| Lw534 | 73 | 628.39 | IEVIR (SEQ ID No:288) |
| | | 748.43 | GTIFRR (SEQ ID No:289) |
| | | 909.42 | GGYEDTLR (SEQ ID No:290) |
| | | 930.51 | TGGLDISIR (SEQ ID No:291) |
| | | 1291.71 | LLDSLALTYGAR (SEQ ID No:292) |
| | | 1370.81 | LLKNTNIILDSK (SEQ ID No:293) |
| | | 1440.70 | FTQNYANLSAANK (SEQ ID No:294) |
| | | 1478.71 | YDNSANQLGTIGAR (SEQ ID No:295) |
| | | 1586.83 | EAAASISVISQNELR (SEQ ID No:296) |
| | | 1604.86 | GMPSAYTLILVDGIR (SEQ ID No:297) |
| | | 1640.87 | LITNASVPQGSGLAGEK (SEQ ID No:298) |
| | | 1654.77 | YEYQTTFGGHISPR (SEQ ID No:299) |
| | | 1705.82 | DASRVESSNTGVELSR (SEQ ID No:300) |
| | | 1707.83 | AYLVWDAQDNWTVK (SEQ ID No:301) |
| | | 1757.91 | LNWNINEQLSTWLK (SEQ ID No:302) |
| | | 1796.97 | LITNASVPQGSGLAGEKR (SEQ ID No:303) |
| | | 1856.01 | IREAAASISVISQNELR (SEQ ID No:451) |
| | | 1912.94 | INSVSIDNTTSTYTNVGK (SEQ ID No:304) |
| | | 2004.03 | DVTLNGAVNNLLDKDFTR (SEQ ID No:305) |
| | | 2072.02 | FSFYSSGPAVEDQLGLSLR (SEQ ID No:306) |
| | | 2155.08 | NKINSVSIDNTTSTYTNVGK (SEQ ID No:307) |
| | | 2301.07 | LDFGTWNSSLSYNQTENIGR (SEQ ID No:308) |
| | | 2395.11 | NYNDLAQALSDVEGVDVNSSTGK (SEQ ID No:309) |
| | | 2484.12 | AWASSATLEHTFQENTAFGDSSK (SEQ ID No:310) |
| | | 2557.36 | WYNNLGSEFKPFSVLNLGVAYK(SEQ ID No:311) |
| | | 2557.36 | WYNNLGSEFKPFSVLNLGVAYK(SEQ ID No:312) |
| | | 2675.42 | TPTLAQLHNGISGVTGQGTITTIGNPK (SEQ ID No:313) |
| | | 2983.33 | DGIVLANNGDEFAQDAWSLFSEDEWR (SEQ ID No:314) |
| | | 3161.51 | THIFAVGNGTTTAGDYFTSSQSTAGYVVPGR (SEQ ID No:315) |
| | | 3184.52 | ITLGNDNRLDFGTWNSSLSYNQTENIGR (SEQ ID No:316) |
| | | 3424.79 | GGVSTGYKTPTLAQLHNGISGVTGQGTITTIGNPK (SEQ ID No:317) |
| | | 3471.62 | LEPESSVNTEVGVYYENETGFGANVTLFHNR (SEQ ID No:318) |
| Lw535 | 64 | 713.42 | VPFVPR (SEQ ID No:319) |
| | | 759.42 | TVGINTR (SEQ ID No:320) |
| | | 773.40 | AATLGDAR (SEQ ID No:321) |
| | | 806.41 | YGALMPR (SEQ ID No:322) |
| | | 919.48 | GPQGTLYGK (SEQ ID No:323) |
| | | 1023.50 | GYIEGGVSSR (SEQ ID No:324) |
| | | 1051.53 | SINYELGTR (SEQ ID No:325) |
| | | 1102.55 | ADATGVELEAK (SEQ ID No:326) |
| | | 1164.56 | DMQLYSGPVR (SEQ ID No:327) |
| | | 1186.57 | WNQDVQELR (SEQ ID No:328) |
| | | 1199.60 | TVDMVFGLYR (SEQ ID No:329) |
| | | 1281.67 | TVGINTRIDFF (SEQ ID No:330) |
| | | 1394.68 | YGAGSSVNGVIDTR (SEQ ID No:331) |
| | | 1444.73 | ADATGVELEAKWR (SEQ ID No:332) |
| | | 1479.70 | ATQDAYVGWNDIK (SEQ ID No:333) |
| | | 1545.80 | TFPSGSLIVNMPQR (SEQ ID No:334) |
| | | 1667.72 | SEFTNDSELYHGNR (SEQ ID No:335) |
| | | 1692.82 | ATQDAYVGWNDIKGR (SEQ ID No:336) |
| | | 1730.85 | FAPGWSWDINGNVIR (SEQ ID No:337) |
| | | 1789.81 | LAPDDQPWEMGFAASR (SEQ ID No:338) |
| | | 1904.85 | TYGYMNGSSAVAQVNMGR (SEQ ID No:339) |
| | | 1968.90 | ECTRATQDAYVGWNDIK (SEQ ID No:340) |
| | | 1981.02 | SAQGGIINIVTQQPDSTPR (SEQ ID No:341) |
| | | 2009.89 | SSTQYHGSMLGNPFGDQGK (SEQ ID No:342) |
| | | 2027.02 | LAVNLVGPHYFDGDNQLR (SEQ ID No:343) |
| | | 2058.99 | YETADVTLQAATFYTHTK (SEQ ID No:344) |
| | | 2162.17 | FNLSGPIQDGLLYGSVTLLR (SEQ ID No:345) |
| | | 2363.13 | SEFTNDSELYHGNRVPFVPR (SEQ ID No:346) |
| | | 2377.30 | SKFNLSGPIQDGLLYGSVTLLR (SEQ ID No:347) |
| | | 2819.49 | VAQGYKPSGYNIVPTAGLDAKPFVAEK (SEQ ID No:348) |
| | | 2929.46 | SASANNVSSTVVSAPELSDAGVTASDKLPR (SEQ ID No:349) |
| Lw536 | 60 | 1010.51 | VEDALHATR (SEQ ID No:350) |
| | | 1186.65 | VAAVKAPGFGDR (SEQ ID No:351) |
| | | 1230.66 | TTLEDLGQAKR (SEQ ID No:352) |
| | | 1237.65 | ARVEDALHATR (SEQ ID No:353) |
| | | 1290.65 | VGAATEVEMKEK (SEQ ID No:354) |
| | | 1566.87 | AAVEEGVVAGGGVALIR (SEQ ID No:355) |
| | | 1604.88 | NVVLDKSFGSPTITK (SEQ ID No:356) |
| | | 1620.85 | SFGSPTITKDGVSVAR (SEQ ID No:357) |
| | | 1668.75 | QQIEDATSDYDKEK (SEQ ID No:358) |
| | | 2020.03 | AAHAIAGLKGDNEDQNVGIK (SEQ ID No:359) |
| | | 2396.29 | VVINKDTTIIIDGVGDEAAIQGR (SEQ ID No:360) |
| Lw537 | 46 | 872.51 | NLSLLSAR (SEQ ID No:361) |
| | | 1000.53 | QTVTTPRAQ (SEQ ID No:362) |
| | | 1179.55 | AAADRDAAYEK (SEQ ID No:363) |
| | | 1257.63 | NNLDNALESLR (SEQ ID No:364) |
| | | 1299.71 | LSQDLAREQIK (SEQ ID No:365) |
| | | 1306.65 | DAAYEKINEVR (SEQ ID No:366) |
| | | 1324.65 | AIDSLSYTEAQK (SEQ ID No:367) |
| | | 1367.75 | TQRPDAVNNLLK (SEQ ID No:368) |
| | | 1394.76 | YNYLINQLNIK (SEQ ID No:369) |
| | | 1435.73 | ASYDTVLAAEVAAR (SEQ ID No:370) |
| | | 1608.93 | LKTQRPDAVNNLLK (SEQ ID No:371) |
| | | 1615.87 | FNVGLVAITDVQNAR (SEQ ID No:372) |
| | | 1779.96 | TILDVLTATTNLYQSK (SEQ ID No:373) |
| | | 1951.01 | QITGVYYPELASLNVER (SEQ ID No:374) |
| | | 1957.97 | AIDSLSYTEAQKQSVYR (SEQ ID No:375) |
| | | 2018.98 | QAQYNFVGASELLESAHR (SEQ ID No:376) |
| | | 2098.10 | SPLLPQLGLSAGYTHANGFR (SEQ ID No:377) |
| | | 2177.16 | QQLADARYNYLINQLNIK (SEQ ID No:378) |
| | | 2709.43 | INEVRSPLLPQLGLSAGYTHANGFR (SEQ ID No:379) |
| Lw538 | 44 | 775.40 | HTPFFK (SEQ ID No:380) |
| | | 836.49 | EHILLGR (SEQ ID No:381) |
| | | 904.49 | FAIREGGR (SEQ ID No:382) |
| | | 1026.58 | AGENVGVLLR (SEQ ID No:383) |
| | | 1072.60 | GTVVTGRVER (SEQ ID No:384) |
| | | 1199.66 | EGGRTVGAGVVAK (SEQ ID No:385) |
| | | 1231.57 | ALEGEAEWEAK (SEQ ID No:386) |
| | | 1232.61 | GYRPQFYFR (SEQ ID No:387) |
| | | 1289.62 | DEGGRHTPFFK (SEQ ID No:388) |
| | | 1375.63 | AFDQIDNAPEEK (SEQ ID No:389) |
| | | 1602.76 | AFDQIDNAPEEKAR (SEQ ID No:390) |
| | | 1613.89 | VGEEVEIVGIKDTVK (SEQ ID No:391) |
| | | 1709.94 | LLDEGRAGENVGVLLR (SEQ ID No:392) |
| | | 1772.87 | GITINTSHVEYDTPAR (SEQ ID No:393) |
| | | 1794.95 | TKPHVNVGTIGHVDHGK (SEQ ID No:394) |
| | | 1904.95 | ELLSAYDFPGDDLPVVR (SEQ ID No:395) |
| | | 1977.01 | IIELAGYLDSYIPEPER (SEQ ID No:396) |
| | | 2000.01 | ARGITINTSHVEYDTPAR (SEQ ID No:397) |
| Lw683 | 37 | 690.4064 | VGFAGLK (SEQ ID No:398) |
| | | 893.4606 | ANAYTGGLK (SEQ ID No:399) |
| | | 910.4330 | GNGMLTYR (SEQ ID No:400) |
| | | 1049.5617 | RANAYTGGLK (SEQ ID No:401) |
| | | 1114.4931 | SSDAAFGFADK (SEQ ID No:402) |
| | | 1119.4906 | NMSTYVDYK (SEQ ID No:403) |
| | | 1121.4697 | NGSSSETNNGR (SEQ ID No:404) |
| | | 1197.5084 | NLDGDQSYMR (SEQ ID No:405) |
| | | 1262.5567 | FADYGSLDYGR (SEQ ID No:406) |
| | | 1307.6146 | IDGLHYFSDNK (SEQ ID No:407) |
| | | 1319.7085 | INLLDKNDFTK (SEQ ID No:408) |
| | | 1422.6739 | TTAQNDLQYGQGK (SEQ ID No:409) |
| | | 1436.6976 | YVDIGATYFFNK (SEQ ID No:410) |
| | | 1490.6022 | AENEDGNHDSFTR (SEQ ID No:411) |
| | | 1533.8038 | GKDIGIYGDQDLLK (SEQ ID No:412) |
| | | 1578.7750 | TTAQNDLQYGQGKR (SEQ ID No:413) |
| | | 2245.1167 | NTNFFGLVDGLNFALQYQGK(SEQ ID No:414) |
| | | 2367.1131 | YDANNVYLAANYTQTYNLTR(SEQ ID No:415) |
| | | 2487.1124 | IDGLHYFSDNKNLDGDQSYMR(SEQ ID No:416) |
| | | 2684.2718 | GETQITDQLTGYGQWEYQVNLNK(SEQ ID No:417) |
| | | 2979.5242 | AHNIEWAQYQFDFGLRPSVAYLQSK(SEQ ID No:418) |
| | | 3292.4764 | GVADQNGDGYGMSLSYDLGWGVSASAAMASSLR(SEQ ID No:419) |
| Lw541 | 31 | 1019.58 | ALASNILYR (SEQ ID No:420) |
| | | 1074.51 | SDPGAAFPWK (SEQ ID No:421) |
| | | 1202.61 | KSDPGAAFPWK (SEQ ID No:422) |
| | | 1247.61 | IFNLVDENER (SEQ ID No:423) |
| | | 1321.58 | MYNIDYNSFR (SEQ ID No:424) |
| | | 1403.64 | AWHAGVSYWDGR (SEQ ID No:425) |
| | | 1786.80 | ALYDAGIGAWYDDETK (SEQ ID No:426) |
| | | 1990.03 | FPDITPVNWGHSDIAPGR (SEQ ID No:427) |
| | | 2090.99 | YGYDTSGAVSEVGYNQLIR (SEQ ID No:428) |
| | | 2118.12 | FPDITPVNVVGHSDIAPGRK(SEQ ID No:429) |
| Lw542 | 31 | 1142.58 | SDPGPLFPWK (SEQ ID No:430) |
| | | 1298.68 | SDPGPLFPWKR (SEQ ID No:431) |
| | | 1307.76 | AIALQLVPEAQR (SEQ ID No:432) |
| | | 1340.64 | AWHAGVSSWQGR (SEQ ID No:433) |
| | | 1370.68 | IPQNGQLDTETR (SEQ ID No:434) |
| | | 1578.77 | GTYQIDTHYPSVAK (SEQ ID No:435) |
| | | 1779.95 | GAASVAVIQQALAAYGYK (SEQ ID No:436) |
| | | 1789.94 | FLVLHYTAVGDAESLR (SEQ ID No:437) |
| | | 1953.00 | YNISPSDVVAHSDIAPLR (SEQ ID No:438) |
| | | 2190.12 | NNLNDTSIGIEIVNLGFTEK (SEQ ID No:439) |
| | | 2630.38 | AIALQLVPEAQRAWHAGVSSWQGR (SEQ ID No:440) |
| Lw544 | 20 | 806.42 | LIDGDFK (SEQ ID No:441) |
| | | 1113.50 | GFEESVDGFK (SEQ ID No:442) |
| | | 1209.60 | VGTWMLGAGYR (SEQ ID No:443) |
| | | 1243.58 | FSSIFGQSESR (SEQ ID No:444) |
| | | 1258.63 | YYSVTAGPVFR (SEQ ID No:445) |
| | | 1269.60 | RGFEESVDGFK (SEQ ID No:446) |
| | | 1356.66 | VGTWMLGAGYRF (SEQ ID No:447) |
| | | 1789.94 | INEYVSLYGLLGAGHGK (SEQ ID No:448) |
| | | 2002.92 | YEFNNDWGVIGSFAQTR (SEQ ID No:449) |
| | | 2988.43 | TSLAYGAGLQFNPHPNFVIDASYEYSK (SEQ ID No:450) |

| | | | |
|---|---|---|---|
| 1. Molecular weight as determined by SDS-PAGE. 2. The mass of a polypeptide fragment can be converted to m/z value by adding 1 to the mass. Each mass includes a range of plus or minus 300 ppm. | | | |

In yet another aspect, the present disclosure further includes polypeptides having similarity with an amino acid sequence. The similarity is referred to as structural similarity and is generally determined by aligning the residues of the two amino acid sequences (i.e., a candidate amino acid sequence and a reference amino acid sequence) to optimize the number of identical amino acids along the lengths of their sequences; gaps in either or both sequences are permitted in making the alignment in order to optimize the number of identical amino acids, although the amino acids in each sequence must nonetheless remain in their proper order. Reference amino acid sequences are disclosed in Table 3 and Table 4. Two amino acid sequences can be compared using commercially available algorithms. Preferably, two amino acid sequences are compared using the Blastp program of the BLAST 2 search algorithm, as described by Tatusova, et al., (FEMSMicrobiol Lett 1999, 174:247-250), and available through the World Wide Web, for instance at the internet site maintained by the National Center for Biotechnology Information, National Institutes of Health. Preferably, the default values for all BLAST 2 search parameters are used, including matrix = BLOSUM62; open gap penalty = 11, extension gap penalty = 1, gap x_dropoff = 50, expect = 10, wordsize = 3, and optionally, filter on. In the comparison of two amino acid sequences using the BLAST search algorithm, structural similarity is referred to as "identities." Preferably, a candidate amino acid sequence has at least 80% identity, at least 90% identity, at least 95% identity, at least 96% identity, at least 97% identity, at least 98% identity, or at least 99% identity to a reference amino acid sequence. Preferably, the molecular weight of the candidate amino acid sequence and the reference amino acid sequence are substantially the same value. Preferably, the molecular weight of the candidate amino acid sequence and the reference amino acid sequence is determined by SDS polyacrylamide gel electrophoresis. A candidate polypeptide can be obtained by growth of a microbe under low metal conditions and the subsequent isolation of a polypeptide by the procedures disclosed herein.

**Table 3.**

| Molecular weight of reference polypeptide (kDa)¹ | NCBI sequence identifier of polypeptide identified by the computer algorithm as having best match to mass fingerprint of reference polypeptide | SEQ ID NO: |
|---|---|---|
| 268 | 23630568, adhesin YadA | 1 |
| 83 | 282049, hemin receptor precursor | 2 |
| 83 | 49114, ferrichrome receptor FcuA | 3 |
| 79 | 565634, ferrioxamine receptor | 4 |
| 70 | 517386, FyuA precursor | 5 |
| 66 | 77958488, Outer membrane receptor for ferrienterochelin and colicins | 6 |
| 45 | 23630568, adhesin YadA | 7 |
| 37 | 77956419, Outer membrane protein (porin) | 8 |
| 31 | 48605, YlpA protein | 9 |

| | | |
|---|---|---|
| 1. Molecular weight as determined by SDS-PAGE. | | |

**Table 4.**

| Molecular weight of reference polypeptide (kDa)¹ | NCBI sequence identifier of polypeptide identified by the computer algorithm as having best match to mass fingerprint of reference polypeptide | SEQ ID NO: |
|---|---|---|
| 104 | 22125915, CoA-linked acetaldehyde dehydrogenase | 10 |
| 99 | 51597993, elongation factor G | 11 |
| 94 | 15981846, glycogen phosphorylase | 12 |
| 88 | 45443416, organic solvent tolerance protein precursor | 13 |
| 77 | 22124457, TonB-dependent outer membrane receptor | 14 |
| 73 | 51595142, putative exogenous ferric siderophore receptor; Iha adhesin | 15 |
| 64 | 22126288, pesticin/yersiniabactin outer membrane receptor | 16 |
| 60 | 51594757, chaperonin GroEL | 17 |
| 46 | 22127390, outer membrane channel precursor protein | 18 |
| 44 | 51597992, elongation factor Tu | 19 |
| 37 | 77633559, Outer membrane protein (porin) | 20 |
| 31 | 22125738, putative regulator | 21 |
| 31 | 22125770, putative regulator | 22 |
| 20 | 22125223, outer membrane protein X | 23 |

| | | |
|---|---|---|
| 1. Molecular weight as determined by SDS-PAGE. | | |

Typically, a candidate amino acid sequence having structural similarity to a reference amino acid sequence has immunogenic activity, protective immunogenic activity, seroactive activity, immunoregulatory activity, or a combination thereof.

The polypeptides expressed by a reference microbe and referred to above by molecular weight can be obtained by growth of the reference microbe under low metal conditions and the subsequent isolation of a polypeptide by the processes disclosed herein. A candidate polypeptide is isolatable from a microbe, preferably a gram negative microbe, more preferably, a member of the family Enterobacteriaceae preferably, a member of the genus *Yersinia,* such as *Y. enterocolitica, Y. pseudotuberculosis,* or *Y. pestis.* A candidate polypeptide may also be produced using recombinant, enzymatic, or chemical techniques.

Also provided by the present disclosure are whole cell preparations of a microbe, where the microbe expresses one or more of the polypeptides of the present disclosure. The cells present in a whole cell preparation are preferably inactivated such that the cells cannot replicate, but the immunogenic activity of the polypeptides of the present disclosure expressed by the microbe is maintained. Typically, the cells are killed by exposure to agents such as glutaraldehyde, formalin, or formaldehyde.

A composition of the present disclosure may include at least one polypeptide described herein, or a number of polypeptides that is an integer greater than 1 (e.g., at least 2, at least 3, at least 4. In some aspects, a composition may include at least 2 metal regulated polypeptides and at least two polypeptides whose expression is not significantly influenced by the presence of a metal. For example, when the polypeptides are isolatable from *Y. enterocolitica,* a composition can include 2, 3, 4, 5, or more isolated metal regulated polypeptides having molecular weights of 268 kDa, 83 kDa , 79 kDa, 70 kDa, 66 kDa, 45 kDa, or any subset or combination thereof, and two isolated polypeptides having a molecular weight of 92 kDa, 54 kDa, 40 kDa, 38 kDa, 37 kDa, 31 kDa, 28 kDa, or any subset or combination thereof. In another example, when the polypeptides are isolatable from *Y. pestis,* a composition can include 2, 3, 4, 5, or more isolated metal regulated polypeptides having molecular weights of 254 kDa, 94 kDa, 88 kDa, 77 kDa, 73 kDa, 64 kDa, 31 kDa, 28 kDa, 20 kDa, or any subset or combination thereof, and two isolated polypeptides having molecular weights of 104 kDa, 99 kDa, 60 kDa, 44 kDa, 46 kDa, 37 kDa, 36 kDa, or any subset or combination thereof. A composition can include polypeptides isolatable from 1 microbe, or can be isolatable from a combination of 2 or more microbes. For instance, a composition can include polypeptides isolatable from 2 or more *Yersinia* spp., from 2 or more *Y. enterocolitica* strains, or from a *Yersinia* spp. and a different microbe that is not a member of the genus *Yersinia.* The present invention also provides compositions comprising an isolated whole cell preparation of a *Yersinia enterocolitica* strain ATCC 27729 or a *Yersinia pestis* KIM6+ strain, as defined in the claims.

Optionally, a polypeptide of the present disclosure can be covalently bound or conjugated to a carrier polypeptide to improve the immunological properties of the polypeptide. Useful carrier polypeptides are known in the art. For example, a polypeptide of the present disclosure could be coupled to known *Yersinia* outer membrane immunogens such as the F1 antigen or the V antigen. Likewise, polysaccharide components could be conjugated to the proteins of the present disclosure to enhance the protective effect of the compositions. The chemical coupling of polypeptides of the present disclosure can be carried out using known and routine methods. For instance, various homobifunctional and/or heterobifunctional cross-linker reagents such as bis(sulfosuccinimidyl) suberate, bis(diazobenzidine), dimethyl adipimidate, dimethyl pimelimidate, dimethyl superimidate, disuccinimidyl suberate, glutaraldehyde, *m*-maleimidobenzoyl-*N* hydroxysuccinimide, sulfo-*m*-maleimidobenzoyl-*N*-hydroxysuccinimide, sulfosuccinimidyl 4-(*N-*maleimidomethyl) cycloheane-1-carboxylate, sulfosuccinimidyl 4-(p-maleimido-phenyl) butyrate and (1-ethyl-3-(dimethylaminopropyl) carbodiimide can be used (see, for instance, Harlow and Lane, Antibodies, A Laboratory Manual, generally and Chapter 5, Cold Spring Harbor Laboratory, Cold Spring Harbor, New York, NY (1988)).

Preferably, such compositions of the present invention include low concentrations of lipopolysaccharide (LPS). LPS is a component of the outer membrane of most gram negative microbes (see, for instance, Nikaido and Vaara, Outer Membrane, In: Escherichia coli and Salmonella typhimurium, Cellular and Molecular Biology, Neidhardt et al., (eds.) American Society for Microbiology, Washington, D.C., pp. 7-22 (1987), and typically includes polysaccharides (O-specific chain, the outer and inner core) and the lipid A region. The lipid A component of LPS is the most biologically active component of the LPS structure and together induce a wide spectrum of pathophysiological effects in mammals. The most dramatic effects are fever, disseminated intravascular coagulation, complement activation, hypotensive shock, and death. The non-specific immunostimulatory activity of LPS can enhance the formation of a granuloma at the site of administration of compositions that include LPS.

The concentration of LPS can be determined using routine methods known in the art. Such methods typically include measurement of dye binding by LPS (see, for instance, Keler and Nowotny, Analyt. Biochem., 156, 189 (1986)) or the use of a *Limulus* amebocyte lysate (LAL) test (see, for instance, Endotoxins and Their Detection With the Limulus Amebocyte Lystate Test, Alan R. Liss, Inc., 150 Fifth Avenue, New York, NY (1982)). There are four basic commercially available methods that are typically used with an LAL test: the gel-clot test; the turbidimetric (spectrophotometric) test; the colorimetric test; and the chromogenic test. An example of a gel-clot assay is available under the tradename E-TOXATE (Sigma Chemical Co., St. Louis, MO; see Sigma Technical Bulletin No. 210), and PYROTELL (Associates of Cape Cod, Inc., East Falmouth, MA). Typically, assay conditions include contacting the composition with a preparation containing a lysate of the circulating amebocytes of the horseshoe crab, *Limulus polyphemus.* When exposed to LPS, the lysate increases in opacity as well as viscosity and may gel. About 0.1 milliliter of the composition is added to lysate. Typically, the pH of the composition is between 6 and 8, preferably, between 6.8 and 7.5. The mixture of composition and lysate is incubated for about 1 hour undisturbed at about 37°C. After incubation, the mixture is observed to determine if there was gelation of the mixture. Gelation indicates the presence of endotoxin. To determine the amount of endotoxin present in the composition, dilutions of a standardized solution of endotoxin are made and tested at the same time that the composition is tested. Standardized solutions of endotoxin are commercially available from, for instance, Sigma Chemical (Catalog No. 210-SE), U.S. Pharmacopeia (Rockville, MD, Catalog No. 235503), and Associates of Cape Cod, Inc., (Catalog No. E0005). In general, when a composition of the present invention is prepared by isolating polypeptides from a microbe by a method as described herein (e.g., a method that includes disrupting and solubilizing the cells, and collecting the insoluble polypeptides), the amount of LPS in a composition of the present invention is less than the amount of LPS present in a mixture of the same amount of the microbe that has been disrupted under the same conditions but not solubilized. Typically, the level of LPS in a composition of the present invention is decreased by, in increasing order of preference, at least 50%, at least 60%, at least 70%, at least 80%, or at least 90% relative to the level of LPS in a composition prepared by disrupting, but not solubilizing, the same microbe.

The compositions of the present invention optionally further include a pharmaceutically acceptable carrier. "Pharmaceutically acceptable" refers to a diluent, carrier, excipient, salt, etc, that is compatible with the other ingredients of the composition, and not deleterious to the recipient thereof. Typically, the composition includes a pharmaceutically acceptable carrier when the composition is used as described herein. The compositions of the present invention may be formulated in pharmaceutical preparations in a variety of forms adapted to the chosen route of administration, including routes suitable for stimulating an immune response to an antigen. Thus, a composition of the present invention can be administered via known routes including, for example, oral; parental including intradermal, transcutaneous and subcutaneous, intramuscular, intravenous, intraperitoneal, etc., and topically, such as, intranasal, intrapulmonary, intramammary, intravaginal, intrauterine, intradermal, transcutaneous and rectally etc. It is foreseen that a composition can be administered to a mucosal surface, such as by administration to the nasal or respiratory mucosa (e.g. spray or aerosol), to stimulate mucosal immunity, such as production of secretory IgA antibodies, throughout the animal's body.

A composition of the present invention can also be administered via a sustained or delayed release implant. Implants suitable for use according to the invention are known and include, for example, those disclosed in Emery and Straub (WO 01/37810 (2001)), and Emery et al., (WO 96/01620 (1996)). Implants can be produced at sizes small enough to be administered by aerosol or spray. Implants also include nanospheres and microspheres.

A composition of the present invention may be administered in an amount sufficient to treat certain conditions as described herein. The amount of polypeptides or whole cells present in a composition of the present invention can vary. For instance, the dosage of polypeptides can be between 0.01 micrograms (µg) and 300 mg, typically between 0.1 mg and 10 mg. When the composition is a whole cell preparation, the cells can be present at a concentration of, for instance, 10² bacteria/ml, 10³ bacteria/ml, 10⁴ bacteria/ml, 10⁵ bacteria/ml, 10⁶ bacteria/ml, 10⁷ bacteria/ml, 10⁸ bacteria/ml, or 10⁹ bacteria/ml. For an injectable composition (e.g. subcutaneous, intramuscular, etc.) the polypeptides may be present in the composition in an amount such that the total volume of the composition administered is 0.5 ml to 5.0 ml, typically 1.0-2.0 ml. When the composition is a whole cell preparation, the cells are preferably present in the composition in an amount that the total volume of the composition administered is 0.5 ml to 5.0 ml, typically 1.0-2.0 ml. The amount administered will vary depending on various factors including, but not limited to, the specific polypeptides chosen, the weight, physical condition and age of the animal, and the route of administration. Thus, the absolute weight of the polypeptide included in a given unit dosage form can vary widely, and depends upon factors such as the species, age, weight and physical condition of the animal, as well as the method of administration. Such factors can be determined by one of skill in the art. Other examples of dosages suitable for the invention are disclosed in Emery et al., (U.S. Patent 6,027,736).

The formulations may be conveniently presented in unit dosage form and may be prepared by methods well known in the art of pharmacy. All methods of preparing a composition including a pharmaceutically acceptable carrier include the step of bringing the active compound (e.g., a polypeptide of the disclosure or whole cell of the composition of the present invention) into association with a carrier that constitutes one or more accessory ingredients. In general, the formulations are prepared by uniformly and intimately bringing the active compound into association with a liquid carrier, a finely divided solid carrier, or both, and then, if necessary, shaping the product into the desired formulations.

A composition including a pharmaceutically acceptable carrier can also include an adjuvant. An "adjuvant" refers to an agent that can act in a nonspecific manner to enhance an immune response to a particular antigen, thus potentially reducing the quantity of antigen necessary in any given immunizing composition, and/or the frequency of injection necessary in order to generate an adequate immune response to the antigen of interest. Adjuvants may include, for example, IL-1, IL-2, emulsifiers, muramyl dipeptides, dimethyldiocradecylammonium bromide (DDA), avridine, aluminum hydroxide, oils, saponins, alpha-tocopherol, polysaccharides, emulsified paraffins (including, for instance, those available from under the tradename EMULSIGEN from MVP Laboratories, Ralston, Nebraska), ISA-70, RIBI and other substances known in the art. It is expected that polypeptides of the present disclosure will have immunoregulatory activity, and that such polypeptides may be used as adjuvants that directly act as T and/or B cell activators or act on specific cell types that enhance the synthesis of various cytokines or activate intracellular signaling pathways. Such polypeptides are expected to augment the immune response to increase the protective index of the existing composition.

In another embodiment, a composition of the invention including a pharmaceutically acceptable carrier can include a biological response modifier, such as, for example, IL-2, IL-4 and/or IL-6, TNF, IFN-alpha, IFN-gamma, and other cytokines that effect immune cells. An immunizing composition can also include other components known in the art such as an antibiotic, a preservative, an antioxidant, or a chelating agent.

The present disclosure also provides methods for obtaining the polypeptides described herein. The polypeptides of the present disclosure and whole cells of composition of the present invention are isolatable from a *Yersinia* spp. Preferred examples include *Y. enterocolitica, Y. pestis,* and *Y. pesudotuberculosis.* Microbes useful for obtaining polypeptides of the present disclosure and making whole cell preparations of the present invention are readily available. In the context of the present invention, whole cells are isolated from a *Yersinia enterocolitica* strain ATCC 27729 or from a *Yersinia pestis* strain KIM6+. In addition, such microbes are readily isolatable by techniques routine and known in the art. The microbes may be derived from an infected animal as a field isolate, and used to obtain polypeptides of the present disclosure and/or whole cell preparations of the present invention, or stored for future use, for example, in a frozen repository at -20°C to -95°C, in bacteriological media containing 20% glycerol, and other like media.

When a polypeptide of the present disclosure is to be obtained from a microbe, the microbe can be incubated under low metal conditions. As used herein, the phrase "low metal conditions" refers to an environment, typically bacteriological media, which contains amounts of a free metal that cause a microbe to express or enhance expression of metal regulated polypeptides. As used herein, the phrase "high metal conditions" refers to an environment that contains amounts of a free metal that cause a microbe to either not express one or more of the metal regulated polypeptides described herein at a detectable level, or to decrease expression of such a polypeptide. Metals are those present in the periodic table under Groups 1 through 17 (IUPAC notation; also referred to as Groups I-A, II-A, III-B, IV-B, V-B, VI-B, VII-B, VIII, I-B, II-B, III-A, IV-A, V-A, VI-A, and VII-A, respectively, under CAS notation). Preferably, metals are those in Groups 2 through 12, more preferably, Groups 3-12. Even more preferably, the metal is iron, zinc, copper, magnesium, nickel, cobalt, manganese, molybdenum, or selenium, most preferably, iron.

Low metal conditions are generally the result of the addition of a metal chelating compound to a bacteriological medium, or the use of bacteriological media formulated to contain low amounts of a metal. High metal conditions are generally present when a chelator is not present in the medium, a metal is added to the medium, or the combination thereof. Examples of metal chelators include natural and synthetic compounds. Examples of natural compounds include plant phenolic compounds, such as flavenoids. Examples of flavenoids include the copper chelators catechin and naringenin, and the iron chelators myricetin and quercetin. Examples of synthetic copper chelators include, for instance, tetrathiomolybdate, and examples of synthetic zinc chelators include, for instance, N,N,N',N' -Tetrakis (2-pyridylmethyl)-ethylene diamine. Examples of synthetic iron chelators include 2,2'-dipyridyl (also referred to in the art as ∀,∀'-bipyridyl), 8-hydroxyquinoline, ethylenediamine-di-O-hydroxyphenylacetic acid (EDDHA), desferrioxamine methanesulphonate (desferol), transferrin, lactoferrin, ovotransferrin, biological siderophores, such as, the catecholates and hydroxamates, and citrate. Preferably, 2,2'-dipyridyl is used for the chelation of iron. Typically, 2,2'-dipyridyl is added to the media at a concentration of at least 0.0025 micrograms/milliliter (µg/ml), at least 0.025 µg/ml, or at least 0.25 µg/ml, and generally no greater than 10 µg/ml, no greater than 20 µg/ml, or no greater than 30 µg/ml.

It is expected that a *Yersinia* spp. with a mutation in *a fur* gene will result in the constitutive expression of many, if not all, of the iron regulated polypeptides of the present disclosure. The production of a *fur* mutation in a *Yersinia* spp. can be produced using routine methods including, for instance, transposon, chemical, or site-directed mutagenesis useful for generating gene knock-out mutations in gram negative bacteria.

The medium used to incubate the microbe and the volume of media used to incubate the microbe can vary. When a microbe is being evaluated for the ability to produce one or more of the polypeptides described herein, the microbe can be grown in a suitable volume, for instance, 10 milliliters to 1 liter of medium. When a microbe is being grown to obtain polypeptides for use in, for instance, administration to animals, the microbe may be grown in a fermentor to allow the isolation of larger amounts of polypeptides. Methods for growing microbes in a fermentor are routine and known in the art. The conditions used for growing a microbe preferably include a metal chelator, more preferably an iron chelator, for instance 2,2'-dipyridyl, a pH of between 6.5 and 7.5, preferably between 6.9 and 7.1, and a temperature of 37°C.

In some aspects of the disclosure, a microbe may be harvested after growth. Harvesting includes concentrating the microbe into a smaller volume and suspending in a media different than the growth media. Methods for concentrating a microbe are routine and known in the art, and include, for example, filtration or centrifugation. Typically, the concentrated microbe is suspended in decreasing amounts of buffer. Preferably, the final buffer includes a cation chelator, preferably, ethylenediaminetetraacetic acid (EDTA). An example of a buffer that can be used contains Tris-base (7.3 grams /liter) and EDTA (0.9 grams/liter), at a pH of 8.5. Optionally, the final buffer also minimizes proteolytic degradation. This can be accomplished by having the final buffer at a pH of greater than 8.0, preferably, at least 8.5, and/or including one or more proteinase inhibitors (e.g., phenylmethanesulfonyl fluoride). Optionally and preferably, the concentrated microbe is frozen at -20°C or below until disrupted.

When the microbe is to be used as a whole cell preparation, the harvested cells may be processed using routine and known methods to inactivate the cells. Alternatively, when a microbe is to be used to prepare polypeptides of the present disclosure, the microbe may be disrupted using chemical, physical, or mechanical methods routine and known in the art, including, for example, french press, sonication, or homogenization. Preferably, homogenization is used. An example of a suitable device useful for homogenization is a model C500 Avestin Homogenizer, (Avestin Inc, Ottawa Canada). As used herein, "disruption" refers to the breaking up of the cell. Disruption of a microbe can be measured by methods that are routine and known in the art, including, for instance, changes in optical density. Typically, a microbe is subjected to disruption until the percent transmittance is increased by 20% when a 1:100 dilution is measured. The temperature during disruption is typically kept low, preferably at 4°C, to further minimize proteolytic degradation.

The disrupted microbe is solubilized in a detergent, for instance, an anionic, zwitterionic, nonionic, or cationic detergent. Preferably, the detergent is sarcosine, more preferably, sodium lauroyl sarcosinate. As used herein, the term "solubilize" refers to dissolving cellular materials (e.g., polypeptides, nucleic acids, carbohydrates) into the aqueous phase of the buffer in which the microbe was disrupted, and the formation of aggregates of insoluble cellular materials. The conditions for solubilization preferably result in the aggregation of polypeptides of the present disclosure into insoluble aggregates that are large enough to allow easy isolation by, for instance, centrifugation.

Significant decreases in LPS are typically observed when the disrupted microbe is solubilized in higher levels of sarcosine, solubilized for longer periods, or the combination thereof. Preferably, the sarcosine is added such that the final ratio of sarcosine to gram weight of disrupted microbe is between 1.0 gram sarcosine per 4.5 grams pellet mass and 6.0 grams sarcosine per 4.5 grams pellet mass, preferably, 4.5 gram sarcosine per 4.5 grams pellet mass. The solubilization of the microbe may be measured by methods that are routine and known in the art, including, for instance, changes in optical density. Typically, the solubilization is allowed to occur for at least 24 hours, preferably, at least 48 hours, more preferably, at least 72 hours, most preferably, at least 96 hours. The temperature during disruption is typically kept low, preferably at 4°C.

The insoluble aggregates that include one or more of the polypeptides of the present disclosure may be isolated by methods that are routine and known in the art. Preferably, the insoluble aggregates are isolated by centrifugation. Typically, centrifugation of polypeptides that are insoluble in detergents requires centrifugal forces of at least 50,000 x g, typically 100,000 x g. The use of such centrifugal forces requires the use of ultracentrifuges, and scale-up to process large volumes of sample is often difficult and not economical with these types of centrifuges. The methods described herein provide for the production of insoluble aggregates large enough to allow the use of significantly lower centrifugal forces (for instance, 46,000 x g). Methods for processing large volumes at these lower centrifugal forces are available and known in the art. Thus, the insoluble aggregates can be isolated at a significantly lower cost. Examples of suitable devices useful for centrifugation of large volumes include T-1 Sharples, (Alfa Laval Separations, Warminster, PA) and Hitachi Himac CC40 high speed centrifuges (Hitachi-Koki Co, Tokyo, Japan).

Optionally and preferably, the sarcosine is removed from the isolated polypeptides. Methods for removing sarcosine from the isolated polypeptides are known in the art, and include, for instance, diafiltration, precipitation, hydrophobic chromatography, ion-exchange chromatography, or affinity chromatography, and ultra filtration and washing the polypeptides in alcohol by diafiltration. After isolation, the polypeptides suspended in buffer and stored at low temperature, for instance, -20°C or below.

Polypeptides of the present disclosure may also be obtained from members of the genus *Yersinia* using methods that are known in the art. The isolation of the polypeptides may be accomplished as described in, for instance, Emery et al., (U.S. Patent 5,830,479) and Emery et al., (U.S. Patent Application US 20030036639 A1).

In those aspects of the present invention where a whole cell preparation is to be made, methods known in the art can be used. For instance, after growth a microbe can be killed with the addition of an agent such as glutaraldehyde, formalin, or formaldehyde, at a concentration sufficient to inactivate the cells in the culture. For instance, formalin can be added at a concentration of 3% (vol:vol). After a period of time sufficient to inactivate the cells, the cells can be harvested by, for instance, diafiltration and/or centrifugation, and washed.

An aspect of the present disclosure is further directed to methods of using the compositions of the present invention. The methods include administering to an animal an effective amount of a composition of the present invention. The animal can be, for instance, avian (including, for instance, chickens or turkeys), bovine (including, for instance, cattle), caprine (including, for instance, goats), ovine (including, for instance, sheep), porcine (including, for instance, swine), bison (including, for instance, buffalo), a companion animal (including, for instance, cats, dogs, and horses), members of the family Cervidae (including, for instance, deer, elk, moose, caribou, and reindeer), piscine (including, for instance, salmon or trout), crustacean (including, for instance, lobster, crab, or shrimp), members of the family Muridae (including, for instance, rats or mice), or human.

In some aspects, the methods may further include additional administrations (e.g., one or more booster administrations) of the composition to the animal to enhance or stimulate a secondary immune response. A booster can be administered at a time after the first administration, for instance, 1 to 8 weeks, preferably 2 to 4 weeks, after the first administration of the composition. Subsequent boosters can be administered one, two, three, four, or more times annually. Without intending to be limited by theory, it is expected that in some aspects of the present disclosure annual boosters will not be necessary, as an animal will be challenged in the field by exposure to microbes expressing polypeptides present in the compositions having epitopes that are identical to or structurally related to epitopes present on polypeptides of the composition administered to the animal.

In one aspect, the disclosure is directed to methods for making antibody, such as inducing the production of antibody in an animal, or by recombinant techniques. The antibody produced includes antibody that specifically binds at least one polypeptide present in the composition. In this aspect of the disclosure, an "effective amount" is an amount effective to result in the production of antibody in the animal. Methods for determining whether an animal has produced antibodies that specifically bind polypeptides present in a composition of the present disclosure can be determined as described herein. The present disclosure further includes antibody that specifically bind to a polypeptide of the present disclosure, and compositions including such antibodies.

The method may be used to produce antibody that specifically binds polypeptides expressed by a microbe other than the microbe from which the polypeptides of the composition were isolated. As used herein, an antibody that can "specifically bind" a polypeptide is an antibody that interacts with the epitope of the antigen that induced the synthesis of the antibody, or interacts with a structurally related epitope. At least some of the polypeptides present in the compositions of the present invention typically include epitopes that are conserved in the polypeptides of different species of microbes. Accordingly, antibody produced using a composition derived from one microbe is expected to bind to polypeptides expressed by other microbes and provide broad spectrum protection against gram negative organisms. Examples of gram negative microbes to which the antibody may specifically bind are enteropathogens, for instance, members of the family Enterobacteriaceae, preferably, members of the genus *Yersinia.*

The present disclosure is also directed to the use of such antibody to target a microbe expressing a polypeptide of the present disclosure or a polypeptide having an epitope structurally related to an epitope present on a polypeptide of the present disclosure. A compound can be covalently bound to an antibody, where the compound can be, for instance, a toxin. Likewise, such compounds can be covalently bound to a bacterial siderophore, such as yersiniabactin, to target the microbe. The chemical coupling or conjugation of an antibody of the present disclosure or a portion thereof (such as an Fab fragment) can be carried out using known and routine methods.

In one aspect the disclosure is also directed to treating an infection in an animal caused by a gram negative microbe, preferably by a member of the genus *Yersinia.* As used herein, the term "infection" refers to the presence of a gram negative microbe, preferably, a member of the genus *Yersinia,* in an animal's body, which may or may not be clinically apparent. An animal with an infection by member of the genus *Yersinia* that is not clinically apparent is often referred to as an asymptomatic carrier. The method includes administering an effective amount of the composition of the present disclosure to an animal having an infection caused by a member of the genus *Yersinia,* and determining whether the *Yersinia* spp. causing the infection has decreased. Methods for determining whether an infection is caused by a member of the genus *Yersinia* are routine and known in the art.

In another aspect, the present disclosure is directed to methods for treating one or more symptoms of certain conditions in animals such as sheep, cattle, goats, pigs, dogs, birds, rodents and deer that may be caused by infection by a member of the genus *Yersinia.* Examples of conditions caused by *Yersinia* spp. infections include, for instance, diarrhea or enteritis in bovine, ovine, and porcine animals and humans, plague-like illnesses in domestic cats and humans, abortion in cattle and sheep, epididymitis-orchitis in rams, and multiple abscess formation in sheep. Yet another aspect of the present disclosure is directed at treating cold water diseases of fish such as enteric red mouth disease in juvenile fish, particularly in intensive aquaculture of trout and salmon. Treatment of symptoms associated with these conditions can be prophylactic or, alternatively, can be initiated after the development of a condition described herein. As used herein, the term "symptom" refers to objective evidence in a subject of a condition caused by infection by a member of the genus *Yersinia* spp. Symptoms associated with conditions referred to herein and the evaluation of such symptoms are routine and known in the art. Treatment that is prophylactic, for instance, initiated before a subject manifests symptoms of a condition caused by a microbe, is referred to herein as treatment of a subject that is "at risk" of developing the condition. Typically, an animal "at risk" of developing a condition is an animal present in an area where the condition has been diagnosed and/or is likely to be exposed to a *Yersinia* spp. causing the condition. Accordingly, administration of a composition can be performed before, during, or after the occurrence of the conditions described herein. Treatment initiated after the development of a condition may result in decreasing the severity of the symptoms of one of the conditions, or completely removing the symptoms. In this aspect of the disclosure, an "effective amount" is an amount effective to prevent the manifestation of symptoms of a disease, decrease the severity of the symptoms of a disease, and/or completely remove the symptoms.

The present disclosure is also directed to decreasing the colonization by gram negative bacteria, for instance blocking the attachment sites by gram negative bacteria, to tissues of the skeletal system (for instance, bones, cartilage, tendons and ligaments), muscular system, (for instance, skeletal and smooth muscles), circulatory system (for instance, heart, blood vessels, capillaries and blood), nervous system (for instance, brain, spinal cord, and peripheral nerves), respiratory system (for instance, nose, trachea lungs, bronchi, bronchioceles, alveoli), digestive system (for instance, mouth, salivary glands oesophagus liver stomach large and small intestine), excretory system (for instance, kidneys, ureters, bladder and urethra), endocrine system (for instance, hypothalamus, pituitary, thyroid, pancreas and adrenal glands), reproductive system (for instance, ovaries, oviduct, uterus, vagina, mammary glands, testes, and seminal vesicles), lymphatic/immune systems (for instance, lymph, lymph nodes and vessels, mononuclear or white blood cells, such as macrophages, neutrophils, monocytes, eosinophils, basophils, lymphocytes t-and b-cells), and specific cell lineages (for instance, precursor cells, epitheial cells, stem cells), and the like. Preferably, the gram negative bacteria is a member of the genus *Yersinia.* The method includes administering an effective amount of a composition of the present invention to an animal colonized by, or at risk of being colonized by a member of the genus *Yersinia.* In this aspect of the disclosure, an "effective amount" is an amount effective to decrease colonization of the animal by the microbe. Methods for evaluating the colonization of an animal by a microbe are routine and known in the art. For instance, colonization of an animal's intestinal tract by a microbe can be determined by measuring the presence of the microbe in the animal's feces. It is expected that decreasing the colonization of an animal by a microbe will reduce transmission of the microbe to humans.

A composition of the invention can be used to provide for active or passive immunization against bacterial infection. Generally, the composition can be administered to an animal to provide active immunization. However, the composition can also be used to induce production of immune products, such as antibodies, which can be collected from the producing animal and administered to another animal to provide passive immunity. Immune components, such as antibodies, can be collected to prepare antibody compositions from serum, plasma, blood, colostrum, etc. for passive immunization therapies. Antibody compositions comprising monoclonal antibodies and/or anti-idiotypes can also be prepared using known methods. Such antibody compositions include chimeric antibodies and humanized antibodies. Chimeric antibodies include human-derived constant regions of both heavy and light chains and murine-derived variable regions that are antigen-specific (Morrison et al., Proc. Natl. Acad. Sci. USA, 1984, 81(21):6851-5; LoBuglio et al., Proc. Natl. Acad. Sci. USA, 1989, 86(11):4220-4; Boulianne et al., Nature, 1984, 312(5995):643-6.). Humanized antibodies substitute the murine constant and framework (FR) (of the variable region) with the human counterparts (Jones et al., Nature, 1986, 321(6069):522-5; Riechmann et al., Nature, 1988, 332(6162):323-7; Verhoeyen et al., Science, 1988, 239(4847):1534-6; Queen et al., Proc. Natl. Acad. Sci. USA, 1989, 86(24):10029-33; Daugherty et al., Nucleic Acids Res., 1991, 19(9): 2471-6.). Alternatively, certain mouse strains can be used that have been genetically engineered to produce antibodies that are almost completely of human origin; following immunization the B cells of these mice are harvested and immortalized for the production of human monoclonal antibodies (Bruggeman and Taussig, Curr. Opin. Biotechnol., 1997, 8(4):455-8; Lonberg and Huszar, Int. Rev. Immunol., 1995;13(1):65-93; Lonberg et al., Nature, 1994, 368:856-9; Taylor et al., Nucleic Acids Res., 1992, 20:6287-95.). Passive antibody compositions and fragments thereof, e.g., scFv, Fab, F(ab')₂ or Fv or other modified forms thereof, may be administered to a recipient in the form of serum, plasma, blood, colostrum, and the like. However, the antibodies may also be isolated from serum, plasma, blood, colostrum, and the like, using known methods for later use in a concentrated or reconstituted form such as, for instance, lavage solutions, impregnated dressings and/or topical agents and the like. Passive immunizing preparations may be particularly advantageous for treatment of acute systemic illness, or passive immunization of young animals that failed to receive adequate levels of passive immunity through maternal colostrum. Antibodies useful for passive immunization may also be useful to conjugate to various drugs or antibiotics that could be directly targeted to bacteria expressing during a systemic or localized infection a polypeptide of the present disclosure or a polypeptide having an epitope structurally related to an epitope present on a polypeptide of the present disclosure.

Animal models, in particular mouse models, are available for experimentally evaluating the compositions of the present invention (see, for instance, Alpar, H. O., et al., Adv. Drug Deliv. Rev., 51, 173-201, (2001), Brem, D., et al., Microbiology, 147, 1115-1127, (2001), Carter, P. B. and F. M. Collins, Infect. Immun., 9, 851-857, (1974), Collyn, F., et al., Infect. Immun., 72, 4784-9470, (2004), Di Genaro, M. S., et al., Microbiol. Immunol., 42, 781-788, (1998), Grosfeld, H., et al., Infect Immun, 71, 374-383, (2003), Jones, S. M., et al., Vaccine, 19, 358-366, (2001), Karlyshev, A. V., et al., Infect Immun, 69, 7810-7819, (2001), Leary, S. E., et al., Microb Pathog, 23, 167-179, (1997), Noll, A., et al., Eur J Immunol, 29, 986-996, (1999), Pelludat, C., et al., Infect Immun, 70, 1832-1841, (2002), Sabhnani, L., et al., FEMS Immunol Med Microbiol, 38, 215-29, (2003), and Williamson, E. D., et al., Vaccine, 19, 566-571, (2000)). These mouse models are commonly accepted models for the study of human disease caused by members of the genus *Yersinia,* and additionally have served as accepted models in the development and initial testing of vaccines aimed at preventing human illnesses by *Yersinia* spp.

Another aspect of the present disclosure provides methods for detecting antibody that specifically binds polypeptides of the present disclosure. These methods are useful in, for instance, detecting whether an animal has antibody that specifically bind polypeptides of the present disclosure, and diagnosing whether an animal may have a condition caused by a microbe expressing polypeptides described herein, or expressing polypeptides that share epitopes with the polypeptides described herein. Such diagnostic systems may be in kit form. The methods include contacting an antibody with a preparation that includes polypeptides of the present disclosure to result in a mixture. The antibody may be present in a biological sample, for instance, blood, milk, or colostrum. The method further includes incubating the mixture under conditions to allow the antibody to specifically bind the polypeptide to form a polypeptide:antibody complex. As used herein, the term "polypeptide:antibody complex" refers to the complex that results when an antibody specifically binds to a polypeptide. The preparation that includes the polypeptides of the present disclosure may also include reagents, for instance a buffer, that provide conditions appropriate for the formation of the polypeptide:antibody complex. The polypeptide:antibody complex is then detected. The detection of antibodies is known in the art and can include, for instance, immunofluorescence and peroxidase. The methods for detecting the presence of antibodies that specifically bind to polypeptides of the present disclosure can be used in various formats that have been used to detect antibody, including radioimmunoassay and enzyme-linked immunosorbent assay.

The present disclosure also provides a kit for detecting antibody that specifically binds polypeptides of the present disclosure. The antibody detected may be obtained from an animal suspected to have an infection caused by a gram negative microbe, more preferably, a member of the family Enterobacteriaceae preferably, a member of the genus *Yersinia,* such as *Y. enterocolitica, Y. pseudotuberculosis,* or *Y. pestis.* The kit includes at least one of the polypeptides of the present disclosure, or a number of polypeptides that is an integer greater than 1 (e.g., at least 2, at least 3, etc.), in a suitable packaging material in an amount sufficient for at least one assay. Optionally, other reagents such as buffers and solutions needed to practice the disclosure are also included. For instance, a kit may also include a reagent to permit detection of an antibody that specifically binds to a polypeptide of the present disclosure, such as a detectably labeled secondary antibody designed to specifically bind to an antibody obtained from an animal. Instructions for use of the packaged polypeptides are also typically included. As used herein, the phrase "packaging material" refers to one or more physical structures used to house the contents of the kit. The packaging material is constructed by well known methods, generally to provide a sterile, contaminant-free environment. The packaging material may have a label which indicates that the polypeptides can be used for detecting antibody that specifically binds polypeptides of the present disclosure. In addition, the packaging material contains instructions indicating how the materials within the kit are employed to detect the antibody. As used herein, the term "package" refers to a container such as glass, plastic, paper, foil, and the like, capable of holding within fixed limits the polypeptides, and other reagents, for instance a secondary antibody. Thus, for example, a package can be a microtiter plate well to which microgram quantities of polypeptides have been affixed. A package can also contain a secondary antibody. "Instructions for use" typically include a tangible expression describing the reagent concentration or at least one assay method parameter, such as the relative amounts of reagent and sample to be admixed, maintenance time periods for reagent/sample admixtures, temperature, buffer conditions, and the like.

The present invention is illustrated by the following examples. To the extent that any of the examples contain subject matter that falls outside the scope of the present invention, they are included merely for reference purposes.

### EXAMPLES

### Example 1

### Production and Isolation of Metal Regulated Proteins

The compositions used in the following examples were prepared using the proteins derived from *Y. enterocolitica* ATCC strain 27729 and *Y. pestis* strain KIM6+ (obtained from R.D. Perry, University of Kentucky). The two strains were each inoculated from frozen stocks into 25 ml tryptic soy broth (TSB) containing 160 µM 2,2-diprydyl or 300 µM FeCl₃, and incubated at 37°C while shaking at 400 rpm. Following 12 hours of incubation, 5 ml of each culture was transferred into 500 ml of pre-incubated (37°C) media containing 160 µM 2,2-diprydyl or 300 µM FeCl₃ and incubated at 37°C while stirring at 100 rpm. After 8 hours of incubation, the cultures were centrifuged at 10,000 x g for 20 minutes. The bacterial pellets were resuspended in 100 ml of sterile physiological saline and centrifuged at 10,000 x g for 10 minutes to remove any contaminating media proteins. The bacterial pellets were then resuspended in 40 ml of Tris-buffered saline pH 7.2 (TBS) and disrupted by sonication for 1.5 minutes at 4°C using a Branson 450 equipped with a half inch disruption horn (Branson, Danbury CT). The disrupted bacterial suspensions were clarified by centrifugation at 32,000 x g for 12 minutes. The supernatants were collected and solubilized by the addition of sodium lauroyl sarcosinate (4% vol/vol) at 4°C for 24 hours. The detergent-insoluble protein-enriched fractions were collected by centrifugation at 32,000 x g for 2.5 hours at 4°C. The protein pellets were resuspended in 200 µl Tris-buffer (pH 7.2) and stored at -90°C. A sample of each extract was resolved on a 10% SDS-PAGE gel per standard methods and visualized by Coomassie Blue staining (Fig. 3).

### Example 2

### Preparation of the immunizing compositions derived from Y. enterocolitica

The proteins made from *Y. enterocolitica* as described in Example 1 were used to prepare a composition for administration to animals. The composition contained polypeptides having molecular weights of 268 kDa, 92 kDa, 83 kDa, 79 kDa, 70 kDa, 66 kDa, 54 kDa, 45 kDa, 40 kDa, 38 kDa, 37 kDa, 31 kDa, or 28 kDa. The polypeptides having molecular weights of 83 kDa, 70 kDa, and 66 kDa were expressed only under iron limited conditions, and the expression of polypeptides having molecular weights of 268 kDa, 79 kDa, and 45 kDa was enhanced under iron limited conditions.

A stock vaccine was prepared from the composition by emulsifying the aqueous protein suspension (500 µg total protein/ml) into the commercial adjuvant, EMULSIGEN, (MVP Laboratories, Ralston, Nebraska) using an IKA Ultra Turrax T-50 homogenizing vessel (IKA, Cincinnati, OH). The vaccine was administered to mice to give a final dose of 50 µg total protein in a 0.1 ml injectable volume with an adjuvant concentration of 22.5% vol/vol. A placebo was prepared by replacing the antigen with physiological saline in the above formulation and emulsifying the suspension into EMULSIGEN to give an adjuvant concentration of 22.5%.

### Example 3

### Preparation of Challenge organism

When used as a challenge, the *Y. enterocolitica* ATCC strain 27729 was prepared as follows. Briefly, the isolate from a frozen stock was streaked onto a blood agar plate and incubated at 37°C for 18 hours. A single colony was subcultured into 50 ml Tryptic Soy Broth (Difco) containing 25 µg/ml 2, 2' dipyridyl. The culture was incubated at 37°C for 6 hours while rotating at 200 rpm, at which point the culture was centrifuged at 10,000 x g for 10 minutes at 4°C to pellet the bacteria. The bacterial pellet was washed twice by centrifugation in physiological saline at 4°C. The final pellet was resuspended in 25 ml of physiological saline and used for challenge. Just prior to challenge, 1ml of the above bacterial suspension was serially diluted ten fold to enumerate the number of CFU/mouse dose.

### Example 4

### Mouse vaccination and challenge study to evaluate protection against intravenous challenge

The efficacy of the *Y. enterocolitica* composition was evaluated using a live virulent challenge in mice. Twenty CF-1 mice (Harlan Breeding Laboratories, Indianapolis, IN) were divided into two groups of 10 mice per group. Mice in the control group were vaccinated with the placebo, while mice in the second group were immunized with 50 µg of the composition obtained as described in Example 1. Immunizations of 0.1 cc were administered intraperitoneally two times at 14 day intervals. Fourteen days after the second vaccination, a challenge dose of strain 27729 (9.4 x 10⁴ CFU/mouse) was administered to all mice in the lateral tail vein. Mortality was recorded for 7 days following challenge.

Of the 10 placebo-vaccinated mice, 10 (100%) died within 168 hours of challenge, while none of the vaccinated mice died within the same time period. Furthermore, all of the vaccinated mice survived for the remainder of the study, which was terminated at 20 days post-challenge. A Kaplan-Meier survival analysis and logrank test (see Figure 1) indicated that immunization provided statistically significant (*p*<0.0001-) protection against challenge. These results suggest that proteins from *Y. enterocolitica* grown under iron-restricted conditions constitute effective antigens in the intravenous mouse model of infection.

### Example 5

### Western blot analysis of Y. enterocolitica proteins with hyperimmunized and convalescent mouse polyclonal serum

Western blot analysis was used to evaluate the immuno-reactive proteins derived from *Y. enterocolitica* against hyperimmunized mouse polyclonal serum and convalescent sera. Hyperimmunizzed mouse polyclonal serum was obtained after vaccinating with the composition described in example 2, and convalescent sera was obtained from vaccinated/challenged mice that survived the trial descrived in example 4. The composition contained polypeptides having molecular weights of 268 kDa, 92 kDa, 79 kDa, 70 kDa, 66 kDa, 54 kDa, 52 kDa, 41 kDa, 38 kDa, 37 kDa, 31 kDa, 28 kDa, and two proteins having molecular weights of 83 kDa. The polypeptides having molecular weights of 83 kDa, 70 kDa, and 66kDa were expressed only under iron limited conditions.

To obtain hyper-immunized serum, mice were immunized two times at 14 day intervals as described in Example 4. The hyperimmunized polyclonal serum was collected from mice 14 days following the second immunization. Convalescent serum derived from vaccinated/challenged mice was obtained 14 days after challenge. The proteins derived from *Y. enterocolitica* strain 27729 were first size fractionated on SDS-PAGE (4 % stacker/10 % resolving gel) using 30 ug total protein as described in example 1. Band migration was visualized using broad range kaleidoscope standards (BioRad) to aid in the electroblot transfer while biotinylated broad range standards were used as molecular weight references on the blot. For western blot analysis, proteins were electroblotted from the SDS-PAGE gel onto trans-blot nitrocellulose membranes (BioRad) overnight, at 4°C at 50 Volts, in Towbin buffer (25mM Tris, 192mM glycine, and 20 % methanol) using a BioRad Trans-Blot transfer cell. The nitrocellulose membrane was blocked by standard methods using 3.0 % fish gelatin (BioRad). The hyperimmunized polyclonal serum and convalescent sera was diluted 1/25000 in Tris-buffered saline containing 1.0 % fish gelatin, 0.05% tween 20 and 0.2% sodium azide (antibody buffer). The nitrocellulose membrane was incubated with the primary antibody solution overnight. The membrane was then washed two times in Tris-Buffered Saline containing 0.05 % tween 20 (TTBS) and transferred to antibody buffer containing a 1/10,000 dilution of goat anti-mouse antibody conjugated to alkaline phosphatase (BioRad) and a 1/3,000 dilution of avidin conjugated to alkaline phosphatase (BioRad). The membrane was incubated at 37°C for 2 hours on a shaker, and subsequently washed in TTBS four times to remove unbound conjugate. The blot was resolved, for 30 minutes at 37°C on a shaker, in substrate solution containing alkaline phosphate color reagent A and B in 1X AP color development buffer (BioRad).

Western blot analysis was used as a tool to potentially identify proteins derived from the composition as described in example 1 as immuno-reactive with antibodies derived from the hyperimmunized and/or convalescent sera. Western blot analysis revealed a number of immuno-reactive proteins. The hyperimmunized sera contained antibodies that reacted with proteins at the 268 kDa, 92 kDa, 83 kDa, 79 kDa, 70 kDa, 66 kDa, 54 kDa, 52 kDa, 41 kDa, 38 kDa, 37 kDa, 31 kDa and 28 kDa. Similarly, the convalescent sera showed identical banding patterns at the 268 kDa, 92 kDa, 83 kDa, 79 kDa, 70 kDa, 66 kDa, 54 kDa, 52 kDa, 41 kDa, 38 kDa, 37 kDa, 31 kDa and 28 kDa. In addition, three immuno-reactive proteins were seen at the 52 kDa, 40 kDa and 20 kDa regions that were not seen on the SDS-PAGE gel initially, nor were they seen in the western blot analysis using the hyperimmunized sera. It is interesting to speculate that these three proteins were at too low of concentration to be visualized on the SDS-PAGE gel, but may be highly immunogenic resulting in greater band intensity after priming the immune system that resulted in an enhanced band intensity of these proteins after challenge.

The Western Blot analysis of the vaccine composition revealed differences in band intensities of the immuno-reactive proteins between both the hyperimmunized and convalescent sera. These differences could be the result of different immunogenic properties of individual proteins and how the immune system recognizes each individual protein within the composition. In addition, the amount and ratio of proteins within the composition can also influence the immunological status of each protein which can influence the immunological response of the animal to individual proteins within the composition. Nevertheless, each protein within the composition reacted immunologically as examined by Western Blot Analysis, thus the immunological response of the mouse upon vaccination, recognized and responded mounting an antibody response to each individual protein within the composition. Taken together, the results as described in example 4 illustrate that the protein composition was extremely efficacious providing a 100 % protection in challenged mice compared to the non-vaccinated mice having 100 % mortality.

### Example 6

### Western blot analysis of Y. pestis proteins with hyperimmunized serum prepared against proteins of Y. enterocolitica

Western blot analysis was used to evaluate the immuno-reactive proteins derived from *Y. pestis* against hyperimmunized sera prepared against the composition derived from *Y. enterocolitica* as described in example 5. The composition contained polypeptides having molecular weights of 254 kDa, 104 kDa, 99 kDa, 94 kDa, 88 kDa, 77 kDa, 73 kDa, 64 kDa, 60 kDa, 46 kDa, 44 kDa, 37 kDa 36 kDa, 31 kDa 28 kDa and 20 kDa. The polypeptides having molecular weights of 94 kDa, 88 kDa, 77 kDa, 73 kDa, and 64 kDa were expressed only under iron limited conditions. The proteins derived from *Y. pestis* strain KIM6+ was first size fractionated on SDS-PAGE (4 % stacker/10 % resolving gel) as previously described in example 5 using 30 ug total protein. Western blot analysis was run under identical conditions as described in example 5 except for the following modification; the convalescent sera was not tested against the membrane proteins of *Y. pestis.* The results showed proteins at approximately the 254 kDa, 94 kDa, 88 kDa, 46 kDa, 44 kDa, 37 kDa, 36 kDa and 20 kDa regions to be immuno-reactive with antibodies derived from the hyperimmunized serum prepared against membrane proteins of *Y. enterocolitica.*

### Example 7

### Mouse vaccination and challenge study to evaluate protection against intravenous and pneumonic Y. pestis challenge

Eighty-eight female Swiss-Webster (Harlan Laboratories) weighing 16-22 grams are equally distributed into 4 groups (22 mice/group), designated 1 through 4. The animals are housed in a HEPA-filtered, micro-vent positive air supply animal caging system (BSL3 facility). Food and water are supplied *ad libitum*.

Proteins from *Y. pestis* strain KIM6+ are prepared as described above in example 1, and formulated as a vaccine using aluminum hydroxide as the adjuvant (Rehydagel-HPA, Rheis NJ) at a final concentration of 20% vol/vol and 500 µg total protein/ml. The placebo is prepared by replacing the antigen with PBS while maintaining the same adjuvant concentration. Mice in Groups 1 and 3 are vaccinated intraperitoneally two times at 14 day intervals with 0.1 ml of vaccine containing 50 µg total protein, while mice in Groups 2 and 4 are immunized with the placebo by an identical schedule.

*Y. pestis* strain CO92 is used for challenge, and is prepared in a BSL3 containment facility. Fourteen days after the second vaccination, mice in Groups 1 and 2 are challenged intravenously in the lateral tail vein with 0.1 ml strain CO92 (10³ CFU or approximately 100 LD₅₀ per mouse). Mice in groups 3 and 4 are subjected to an aerosolized challenge dose of *Y. pestis* CO92 diluted in physiological saline to achieve an approximate concentration of 100 LD₅₀ CFU per mouse for 30 minutes in an airtight chamber. The aerosolized LD₅₀ for strain CO92 in Swiss Webster mice is determined by small pilot studies prior to the proposed challenge experiments. Mortality is recorded for 21 days after challenge.

### Example 8

### Fish vaccination and challenge study to evaluate protection against Y. ruckeri challenge

Two groups of 20 rainbow trout, designated as groups 1 and 2 weighing approximately 2 grams are maintained in two separate 60 liter tanks at a temperature of 18°C. Fish are fed twice daily with a commercial trout feed (Ziegler Brothers, Gardners, PA). Fish in group 1 are vaccinated with a composition derived from *Y. ruckeri* using the same method as described in example 1. The extracted proteins derived from *Y. ruckeri* are used to prepare a vaccine composition for administration to fish. A stock vaccine is prepared from the composition by emulsifying the aqueous protein suspension into a water-in-oil emulsion containing Drakeol 6 mineral oil and Arlacel A as an emulsifier. The vaccine is administered intraperitoneally to give a final dose of 25 ug total protein in a 0.1 cc injectable volume using 0.1 cc. A placebo is prepared by replacing the antigen with physiological saline in the above formulation and is given to the fish in group 2 (controls). Fish are given a second vaccination 28 days after the first vaccination. Fourteen days after the second vaccination all fish are intraperitoneally challenged.

A virulent isolate of *Y. ruckeri* is used for challenge. The challenge isolate is cultured in Tryticase Soy Broth (TSB) containing 160 µM 2,2-diprydyl and grown for 12 hours of incubation at 37°C. The culture is washed once in physiological saline by centrifugation at 10,000 x g and resuspended in saline. The culture is adjusted to 5.0 x 10⁷ CFU per ml. Each trout is intraperitoneally inoculated with 0.1 cc of the corresponding bacteria at a final challenge dose of 5.0 x 10⁶ CFU. Mortality was recorded daily for 14 days after challenge. All dead fish are removed from the tank and the livers are removed and plated to enumerate the presence of the challenge organism. Efficacy is measured as a degree of livability comparing vaccinates to non-vaccinated controls.

### Example 9

### Characterization of metal regulated proteins of Y. enterocolitica ATCC strain 27729 and Y. pestis strain KIM6+

The proteins of the composition prepared as described in example 1 from *Y. enterocolitica* ATCC strain 27729 and *Y. pestis* strain KIM6+ were characterized using matrix assisted laser desorption/ionization time-of-flight spectrometry (MALDI-TOF MS). Samples of each composition were was resolved using a 10% sodium dodecyl sulfate-polyacrylamide gel. After the proteins of a composition had been resolved, the gel was stained with coomasie brilliant blue to visualize the proteins.

### Materials and Methods

*Excision and washing.* The gel was washed for 10 minutes with water twice. Each protein band of interest was excised by cutting as close to the protein band as possible to reduce the amount of gel present in the sample. Each gel slice was cut into 1x1 mm cubes and placed in 1.5 ml tube. The gel pieces were washed with water for 15 minutes. All the solvent volumes used in the wash steps were approximately equal to twice the volume of the gel slice. The gel slice was next washed with water/acetonitrile (1:1) for 15 minutes. The water/acetonitrile mixture was removed, and acetonitrile was added to cover until the gel pieces turned a sticky white, at which time the acetonitrile was removed. The gel pieces were rehydrated in 100 mM NH₄HCO₃, and after 5 minutes, a volume of acetonitrile equal to twice the volume of the gel pieces was added. This was incubated for 15 minutes, the liquid removed, and the gel pieces dried in a SpeedVac.

*Reduction & alkylation.* The dried gel pieces were rehydrated in 10mM DTT and 100 mM NH₄HCO₃, and incubated for 45 minutes at 56°C. After allowing the tubes to cool to room temperature, the liquid was removed and the same volume of a mixture of 55mM iodoacetamide and 100 mM NH₄HCO₃ was immediately added. This was incubated for 30 minutes at room temperature in the dark. The liquid was removed, acetonitrile was added to cover until the gel pieces turned a sticky white, at which time the acetonitrile was removed. The gel pieces were rehydrated in 100 mM NH₄HCO₃, and after 5 minutes, a volume of acetonitrile equal to twice the volume of the gel pieces was added. This was incubated for 15 minutes, the liquid removed, and the gel pieces dried in a Speed vac. If residual coomassie still remained, the wash with 100 mM NH₄HCO₃/acetonitrile was repeated.

*In-gel digestion.* Gel pieces were completely dried down in a Speed Vac. The pieces were rehydrated in digestion buffer (50 mM NH₄HCO₃, 5 mM CaCl₂, 12.5 nanograms per microliter (ng/µl) trypsin) at 4°C. Enough buffer was added to cover the gel pieces, and more was added as needed. The gel pieces were incubated on ice for 45 minutes, and the supernatant removed and replaced with 5-2 µl of same buffer without trypsin. This was incubated at 37°C overnight in an air incubator.

*Extraction of peptides.* A sufficient volume of 25 mM NH₄HCO₃ was added to cover gel pieces, and incubated for 15 minutes (typically in a bath sonicator). The same volume of acetonitrile was added and incubated for 15 minutes (in a bath sonicator if possible), and the supernatant was recovered. The extraction was repeated twice, using 5% formic acid instead of NH₄HCO₃. A sufficient volume of 5% formic acid was added to cover gel pieces, and incubated for 15 minutes (typically in a bath sonicator). The same volume of acetonitrile was added and incubated for 15 minutes (typically in a bath sonicator), and the supernatant was recovered. The extracts were pooled, and 10 mM DTT was added to a final concentration of 1 mM DTT. The sample was dried in a SpeedVac to a final volume of approximately 5 µl.

*Desalting of peptides.* The samples were desalted using ZIPTIP pipette tips (C18, Millipore, Billerica, MA) as suggested by the manufacturer. Briefly, a sample was reconstituted in reconstitution solution (5:95 acetonitrile:H₂O, 0.1% - 0.5% trifluoroacetic acid), centrifuged, and the pH checked to verify that it was less than 3. A ZIPTIP was hydrated by aspirating 10 µl of solution 1 (50:50 acetonitrile:H₂O, 0.1% trifluoroacetic acid) and discarding the aspirated aliquots. This was followed by aspirating 10 µl of solution 2 (0.1% trifluoroacetic acid in deionized H₂O) and discarding the aspirated aliquots. The sample was loaded into the tip by aspirating 10 µl of the sample slowly into the tip, expelling it into the sample tube, and repeating this 5 to 6 times. Ten microliters of solution 2 was aspirated into the tip, the solution discarded by expelling, and this process was repeated 5-7 times to wash. The peptides were eluted by aspirating 2.5 µl of ice cold solution 3 (60:40, acetonitrile:H₂O, 0.1% trofluoroacetic acid), expelling, and then re-aspirating the same aliquot in and out of the tip 3 times. After the solution has been expelled from the tip, the tube is capped and stored on ice.

*Mass spectrometric peptide mapping.* The peptides were suspended in 10 µl to 30 µl of 5% formic acid, and analyzed by MALDI-TOF MS (Bruker Daltonics Inc., Billerica, MA). The mass spectrum of the peptide fragments was determined as suggested by the manufacturer. Briefly, a sample containing the peptides resulting from a tryptic digest were mixed with matrix cyano-4-hydroxycinnamic acid, transferred to a target, and allowed to dry. The dried sample was placed in the mass spectrometer, irradiated, and the time of flight of each ion detected and used to determine a peptide mass fingerprint for each protein present in the composition. Known polypeptides (human angiotensin II, monoisotopic mass MH⁺ 1046.5 (Sigma Chemical Co.), and adenocorticotropin hormone fragment 18-39, MH⁺ 2465.2 (Sigma Chemical Co.)) were used to standardize the machine.

*Data analysis.* The experimentally observed masses for the peptides in each mass spectrum were compared to the expected masses of resulting from known proteins using the Peptide Mass Fingerprint search method of the Mascot search engine (Matrix Science Ltd., London, UK, and www.matrixscience.com, see Perkins et al., Electrophoresis 20, 3551-3567 (1999)). The search parameters included: database, NCBInr; taxonomy, bacteria (eubacteria); type of search, peptide mass fingerprint; enzyme, trypsin; fixed modifications, none; variable modifications, none or oxidized methionine; mass values, monoisotopic; protein mass, unrestricted; peptide mass tolerance, ±1 Da or ±1 330 ppm; peptide charge state, Mr; max missed cleavages, 1; number of queries, 25.

### Results

The result of this search was a mass fingerprint for protein present in the composition (Tables 5 and 6).

**Table 5. Experimental data from MALDI-TOF MS analysis of proteins isolated from Y. enterocolitica ATCC strain 27729.**

| Polypeptide Designation | Approximate molecular weight in kilodaltons (kDa)¹ | m/z value of polypeptide fragments resulting from trypsin digestion² |
|---|---|---|
| Lw545 | 268 | 929.46 |
| | | 1140.47 |
| | | 1312.57 |
| | | 1440.69 |
| | | 1526.68 |
| | | 1555.66 |
| | | 1581.70 |
| | | 1596.67 |
| | | 1683.69 |
| | | 2110.21 |
| Lw391A (± 1 Da) | 83 | 687.5 |
| | | 976.4 |
| | | 1001.6 |
| | | 1016.5 |
| | | 1141.6 |
| | | 1170.7 |
| | | 1171.7 |
| | | 1198.5 |
| | | 1344.5 |
| | | 1357.7 |
| | | 1395.6 |
| | | 1453.7 |
| | | 1477.7 |
| | | 1521.7 |
| | | 1693.8 |
| | | 1716.8 |
| | | 1829.8 |
| | | 1962.0 |
| | | 2014.1 |
| | | 2020.0 |
| | | 2042.0 |
| | | 2164.1 |
| | | 2226.1 |
| | | 2417.3 |
| | | 3175.5 |
| Lw391B (± 1 Da) | 83 | 1001.6 |
| | | 1104.6 |
| | | 1140.6 |
| | | 1155.5 |
| | | 1171.7 |
| | | 1209.5 |
| | | 1214.7 |
| | | 1338.6 |
| | | 1453.7 |
| | | 1568.8 |
| | | 1634.9 |
| | | 1651.8 |
| | | 1660.9 |
| | | 1709.8 |
| | | 1750.0 |
| | | 1851.0 |
| | | 1988.1 |
| | | 2105.1 |
| | | 2112.1 |
| | | 2164.1 |
| | | 2387.2 |
| | | 2453.1 |
| | | 2538.4 |
| | | 3423.7 |
| Lw392 (± 1 Da | 79 | 837.5 |
| | | 1018.6 |
| | | 1071.5 |
| | | 1086.5 |
| | | 1132.7 |
| | | 1189.5 |
| | | 1215.6 |
| | | 1236.6 |
| | | 1256.6 |
| | | 1264.6 |
| | | 1361.6 |
| | | 1497.7 |
| | | 1502.8 |
| | | 1615.7 |
| | | 1653.8 |
| | | 1718.9 |
| | | 1770.9 |
| | | 1820.9 |
| | | 1828.1 |
| | | 2006.0 |
| | | 2067.1 |
| | | 2120.9 |
| | | 2300.3 |
| | | 2308.2 |
| | | 2783.3 |
| | | 2912.4 |
| | | 3024.5 |
| | | 3287.6 |
| Lw393 (± 1 Da) | 70 | 714.6 |
| | | 760.5 |
| | | 807.5 |
| | | 820.5 |
| | | 920.5 |
| | | 1024.6 |
| | | 1052.6 |
| | | 1187.6 |
| | | 1200.6 |
| | | 1395.7 |
| | | 1437.7 |
| | | 1480.7 |
| | | 1541.9 |
| | | 1546.9 |
| | | 1565.8 |
| | | 1668.8 |
| | | 1732.0 |
| | | 1790.9 |
| | | 1906.0 |
| | | 1982.2 |
| | | 1984.1 |
| | | 1997.1 |
| | | 2011.1 |
| | | 2028.2 |
| | | 2060.2 |
| | | 2134.1 |
| | | 2163.3 |
| | | 2275.4 |
| | | 2364.3 |
| | | 2378.5 |
| | | 2384.3 |
| | | 2564.4 |
| | | 2658.4 |
| | | 2834.7 |
| | | 2930.7 |
| Lw550 | 66 | 868.6500 |
| | | 882.5700 |
| | | 884.5900 |
| | | 1021.7000 |
| | | 1087.7100 |
| | | 1168.7300 |
| | | 1177.8200 |
| | | 1208.6800 |
| | | 1346.8700 |
| | | 1750.0100 |
| | | 1755.0500 |
| | | 1852.2800 |
| | | 2521.8100 |
| | | 2607.6700 |
| | | 2944.1000 |
| | | 3087.0800 |
| Lw552 | 45 | 1140.5500 |
| | | 1209.5400 |
| | | 1312.5800 |
| | | 1440.6400 |
| | | 1501.6900 |
| | | 1526.6200 |
| | | 1581.6800 |
| | | 1596.6800 |
| Lw555 | 37 | 705.3700 |
| | | 881.2400 |
| | | 971.1700 |
| | | 1122.3100 |
| | | 1280.1900 |
| | | 1295.2200 |
| | | 1335.2900 |
| | | 1510.3000 |
| | | 1908.5300 |
| | | 2245.7300 |
| | | 2324.7100 |
| | | 2642.7500 |
| | | 2985.0200 |
| | | 3087.9700 |
| Lw557 | 31 | 864.49 |
| | | 1404.50 |
| | | 1616.68 |
| | | 1780.68 |
| | | 1876.82 |
| | | 2071.04 |
| | | 2379.08 |

| | | |
|---|---|---|
| 1. Molecular weight, in kilodaltons, of polypeptide obtained from *Y. enterocolitica* ATCC strain 27729. 2. m/z, mass (m) to charge (z) ratio. | | |

**Table 6. Experimental data from MALDI-TOF MS analysis of proteins isolated from Y. pestis strain KIM6+.**

| Polypeptide Designation | Approximate molecular weight in kilodaltons (kDa)¹ | m/z value of polypeptide fragments resulting from trypsin digestion² |
|---|---|---|
| Lw529 | 104 | 644.50 |
| | | 685.40 |
| | | 771.40 |
| | | 841.40 |
| | | 899.50 |
| | | 962.40 |
| | | 1137.40 |
| | | 1277.40 |
| | | 1293.40 |
| | | 1386.40 |
| | | 1410.50 |
| | | 1422.60 |
| | | 1498.60 |
| | | 1567.50 |
| | | 1679.70 |
| | | 1684.60 |
| | | 1726.70 |
| | | 1873.70 |
| | | 1991.70 |
| | | 2020.80 |
| | | 2182.80 |
| | | 2584.90 |
| | | 2843.20 |
| Lw530 | 99 | 1191.40 |
| | | 1514.50 |
| | | 1591.50 |
| | | 1597.50 |
| | | 1637.50 |
| | | 1671.50 |
| | | 1714.60 |
| | | 1719.60 |
| | | 1751.60 |
| | | 1820.60 |
| | | 1863.70 |
| | | 1967.60 |
| | | 2122.60 |
| Lw531 | 94 | 962.20 |
| | | 1168.20 |
| | | 1258.30 |
| | | 1372.30 |
| | | 1384.30 |
| | | 1409.30 |
| | | 1521.40 |
| | | 1669.50 |
| | | 1686.40 |
| | | 1714.40 |
| | | 1717.40 |
| | | 1797.50 |
| | | 1833.50 |
| | | 1845.50 |
| | | 2218.60 |
| | | 2426.60 |
| Lw532 | 88 | 889.30 |
| | | 927.30 |
| | | 946.40 |
| | | 961.40 |
| | | 1172.40 |
| | | 1177.40 |
| | | 1290.40 |
| | | 1333.50 |
| | | 1358.40 |
| | | 1404.50 |
| | | 1419.50 |
| | | 1508.50 |
| | | 1579.60 |
| | | 1673.60 |
| | | 1736.60 |
| | | 2401.00 |
| | | 2666.00 |
| Lw533 | 77 | 687.40 |
| | | 785.40 |
| | | 859.30 |
| | | 953.40 |
| | | 1141.50 |
| | | 1156.50 |
| | | 1171.50 |
| | | 1198.40 |
| | | 1403.50 |
| | | 1409.50 |
| | | 1483.50 |
| | | 1523.50 |
| | | 1551.60 |
| | | 1618.60 |
| | | 1675.50 |
| | | 1746.60 |
| | | 1788.70 |
| | | 1820.70 |
| | | 1852.80 |
| | | 1941.60 |
| | | 2013.90 |
| | | 2018.80 |
| | | 2057.80 |
| | | 2168.80 |
| | | 2170.00 |
| | | 2427.00 |
| | | 2457.80 |
| | | 2829.10 |
| Lw534 | 73 | 629.40 |
| | | 749.40 |
| | | 910.30 |
| | | 931.40 |
| | | 1292.50 |
| | | 1371.50 |
| | | 1441.40 |
| | | 1479.50 |
| | | 1587.60 |
| | | 1605.60 |
| | | 1641.60 |
| | | 1655.50 |
| | | 1706.60 |
| | | 1708.60 |
| | | 1758.70 |
| | | 1797.80 |
| | | 1856.80 |
| | | 1913.70 |
| | | 2004.80 |
| | | 2072.80 |
| | | 2155.90 |
| | | 2301.90 |
| | | 2395.90 |
| | | 2484.90 |
| | | 2558.20 |
| | | 2676.20 |
| | | 2984.10 |
| | | 3162.30 |
| | | 3185.30 |
| | | 3425.50 |
| | | 3472.40 |
| Lw535 | 64 | 714.40 |
| | | 760.40 |
| | | 774.40 |
| | | 807.40 |
| | | 920.40 |
| | | 1024.40 |
| | | 1052.40 |
| | | 1103.40 |
| | | 1165.40 |
| | | 1187.40 |
| | | 1200.40 |
| | | 1282.50 |
| | | 1395.40 |
| | | 1445.50 |
| | | 1480.50 |
| | | 1546.60 |
| | | 1668.50 |
| | | 1693.60 |
| | | 1731.60 |
| | | 1790.60 |
| | | 1905.70 |
| | | 1969.70 |
| | | 1981.80 |
| | | 2010.80 |
| | | 2027.80 |
| | | 2059.80 |
| | | 2163.00 |
| | | 2363.90 |
| | | 2378.10 |
| | | 2820.20 |
| | | 2930.20 |
| Lw536 | 60 | 1011.46 |
| | | 1187.55 |
| | | 1231.54 |
| | | 1238.57 |
| | | 1291.57 |
| | | 1567.76 |
| | | 1605.78 |
| | | 1621.74 |
| | | 1669.68 |
| | | 2021.02 |
| | | 2397.21 |
| Lw537 | 46 | 873.53 |
| | | 1001.53 |
| | | 1180.50 |
| | | 1258.60 |
| | | 1300.67 |
| | | 1307.58 |
| | | 1325.59 |
| | | 1368.72 |
| | | 1395.70 |
| | | 1436.67 |
| | | 1609.91 |
| | | 1616.82 |
| | | 1780.94 |
| | | 1952.05 |
| | | 1959.02 |
| | | 2020.04 |
| | | 2099.15 |
| | | 2178.22 |
| | | 2710.51 |
| Lw538 | 44 | 776.51 |
| | | 837.65 |
| | | 905.62 |
| | | 1027.71 |
| | | 1073.74 |
| | | 1200.79 |
| | | 1232.67 |
| | | 1233.72 |
| | | 1290.81 |
| | | 1376.71 |
| | | 1603.90 |
| | | 1615.01 |
| | | 1711.08 |
| | | 1774.04 |
| | | 1796.13 |
| | | 1906.14 |
| | | 1978.16 |
| | | 2001.23 |
| Lw683 | 37 | 691.26 |
| | | 894.21 |
| | | 911.21 |
| | | 1050.26 |
| | | 1115.19 |
| | | 1120.19 |
| | | 1122.24 |
| | | 1198.17 |
| | | 1263.19 |
| | | 1308.24 |
| | | 1320.34 |
| | | 1423.28 |
| | | 1437.31 |
| | | 1491.23 |
| | | 1534.41 |
| | | 1579.39 |
| | | 2245.71 |
| | | 2367.68 |
| | | 2487.63 |
| | | 2684.79 |
| | | 2980.02 |
| | | 3292.91 |
| Lw541 | 31 | 1020.84 |
| | | 1075.77 |
| | | 1203.86 |
| | | 1248.88 |
| | | 1322.87 |
| | | 1404.95 |
| | | 1788.29 |
| | | 1991.60 |
| | | 2092.61 |
| | | 2119.74 |
| Lw542 | 31 | 1143.91 |
| | | 1299.97 |
| | | 1309.09 |
| | | 1341.97 |
| | | 1372.04 |
| | | 1580.12 |
| | | 1781.45 |
| | | 1791.43 |
| | | 1954.57 |
| | | 2191.78 |
| | | 2632.11 |
| Lw544 | 20 | 807.40 |
| | | 1114.43 |
| | | 1210.48 |
| | | 1244.46 |
| | | 1259.51 |
| | | 1270.49 |
| | | 1357.49 |
| | | 1790.90 |
| | | 2003.91 |
| | | 2989.45 |

| | | |
|---|---|---|
| 1. Molecular weight, in kilodaltons, of polypeptide obtained from *Y. pestis* strain KIM6+. 2. m/z, mass (m) to charge (z) ratio. | | |

## Claims

1. A composition obtainable by a process comprising:
providing a culture comprising a *Yersinia enterocolitica* strain ATCC 27729, wherein the *Yersinia enterocolitica* has been grown at 37°C in tryptic soy broth (TSB) medium comprising 160 µM 2,2'-dipyridyl;
disrupting the *Yersinia enterocolitica* to result in a mixture comprising disrupted cell membranes;
solubilizing the mixture by adding to the mixture a biological detergent to result in a preparation comprising solubilized and unsolubilized proteins; and
isolating the unsolubilized polypeptides.

2. A composition comprising an isolated whole cell preparation of a *Yersinia enterocolitica* strain ATCC 27729, wherein the cells have been grown at 37°C in TSB medium comprising 160 µM 2,2'-dipyridyl.

3. A composition obtainable by a process comprising:
providing a culture comprising a *Yersinia enterocolitica* strain ATCC 27729, wherein the *Yersinia enterocolitica* has been grown at 37°C in TSB medium comprising 160 µM 2,2'-dipyridyl;
inactivating the *Yersinia enterocolitica* to result in a composition comprising inactivated *Yersinia enterocolitica* cells, wherein the inactivating occurs under conditions that do not disrupt the cells; and
harvesting the inactivated cells.

4. A composition obtainable by a process comprising:
providing a culture comprising a *Yersinia pestis* strain KIM6+, wherein the *Yersinia pestis* has been grown at 37°C in TSB medium comprising 160 µM 2,2'-dipyridyl;
disrupting the *Yersinia pestis* to result in a mixture comprising disrupted cell membranes;
solubilizing the mixture by adding to the mixture a biological detergent to result in a preparation comprising solubilized and unsolubilized proteins; and
isolating the unsolubilized polypeptides.

5. A composition comprising an isolated whole cell preparation of a *Yersinia pestis* KIM6+ strain, wherein the cells have been grown at 37°C in TSB medium comprising 160 µM 2,2'-dipyridyl.

6. A composition obtainable by a process comprising:
providing a culture comprising a *Yersinia pestis* KIM6+ strain, wherein the *Yersinia pestis* has been grown at 37°C in TSB medium comprising 160 µM 2,2'-dipyridyl;
inactivating the *Yersinia pestis* to result in a composition comprising inactivated *Yersinia pestis* cells, wherein the inactivating occurs under conditions that do not disrupt the cells; and
harvesting the inactivated cells.

7. A composition according to any of Claims 1-6 for use as a medicament.

## Patentansprüche

1. Zusammensetzung, erhältlich über einen Vorgang, der Folgendes umfasst:
Bereitstellen einer Kultur, umfassend einen *Yersinia-enterocolitica-Stamm* ATCC 27729, wobei die *Yersinia enterocolitica* bei 37 °C in Medium aus tryptischer Sojabrühe (TSB), umfassend 160 µM 2,2'-Dipyridyl, gezüchtet worden ist;
Aufbrechen der *Yersinia enterocolitica,* um ein Gemisch zu ergeben, das aufgebrochene Zellmembranen umfasst;
Solubilisieren des Gemischs durch Hinzufügen eines biologischen Waschmittels zu dem Gemisch, um ein Präparat zu ergeben, das solubilisierte und unsolubilisierte Proteine umfasst; und
Isolieren der unsolubilisierten Polypeptide.

2. Zusammensetzung, die ein isoliertes Gesamtzellpräparat eines *Yersinia-enterocolitica*-Stammes ATCC 27729 umfasst, wobei die Zellen bei 37 °C in TSB-Medium, umfassend 160 µM 2,2'-Dipyridyl, gezüchtet worden sind.

3. Zusammensetzung, erhältlich über einen Vorgang, der Folgendes umfasst:
Bereitstellen einer Kultur, die einen *Yersinia-enterocolitica-Stamm* ATCC 27729 umfasst, wobei die *Yersinia enterocolitica* bei 37 °C in TSB-Medium, umfassend 160 µM 2,2'-Dipyridyl, gezüchtet worden ist;
Deaktivieren der *Yersinia enterocolitica,* um eine Zusammensetzung zu ergeben, die deaktivierte *Yersinia-enterocolitica*-Zellen umfasst, wobei das Deaktivieren unter Bedingungen stattfindet, die die Zellen nicht aufbrechen; und
Ernten der deaktivierten Zellen.

4. Zusammensetzung, erhältlich über einen Vorgang, der Folgendes umfasst:
Bereitstellen einer Kultur, die einen *Yersinia-pestis-Stamm* KIM6+ umfasst, wobei die *Yersinia pestis* bei 37 °C in TSB-Medium, umfassend 160 µM 2,2'-Dipyridyl, gezüchtet worden ist;
Aufbrechen der *Yersinia pestis,* um ein Gemisch zu ergeben, das aufgebrochene Zellmembranen umfasst;
Solubilisieren des Gemischs durch Hinzufügen eines biologischen Waschmittels zu dem Gemisch, um ein Präparat zu ergeben, das solubilisierte und unsolubilisierte Proteine umfasst; und
Isolieren der unsolubilisierten Polypeptide.

5. Zusammensetzung, die ein isoliertes Gesamtzellpräparat eines *Yersinia-pestis-*Stammes KIM6+ umfasst, wobei die Zellen bei 37 °C in TSB-Medium, umfassend 160 µM 2,2'-Dipyridyl, gezüchtet worden sind.

6. Zusammensetzung, erhältlich über einen Vorgang, der Folgendes umfasst:
Bereitstellen einer Kultur, die einen *Yersinia-pestis-Stamm* KIM6+ umfasst, wobei die *Yersinia pestis* bei 37 °C in TSB-Medium, umfassend 160 µM 2,2'-Dipyridyl, gezüchtet worden ist;
Deaktivieren der *Yersinia pestis,* um eine Zusammensetzung zu ergeben, die deaktivierte *Yersinia-pestis-Zellen* umfasst, wobei das Deaktivieren unter Bedingungen stattfindet, die die Zellen nicht aufbrechen; und
Ernten der deaktivierten Zellen.

7. Zusammensetzung nach einem der Ansprüche 1-6 für den Gebrauch als ein Arzneimittel.

## Revendications

1. Composition pouvant être obtenue par un procédé comprenant les étapes consistant à :
fournir une culture comprenant une souche *Yersinia enterocolitica* ATCC 27729, dans laquelle *Yersinia enterocolitica* a été cultivée à 37 °C dans un milieu de type bouillon trypticase soja (TSB) comprenant 160 µM de 2,2'-dipyridyle ;
perturber *Yersinia enterocolitica* pour produire un mélange comprenant des membranes cellulaires perturbées ;
solubiliser le mélange en ajoutant au mélange un détergent biologique pour produire une préparation comprenant des protéines solubilisées et non solubilisées ; et
isoler les polypeptides non solubilisés.

2. Composition comprenant une préparation de cellules entières isolées d'une souche *Yersinia enterocolitica* ATCC 27729, dans laquelle les cellules ont été cultivées à 37 °C dans un milieu TSB comprenant 160 µM de 2,2'-dipyridyle.

3. Composition pouvant être obtenue par un procédé comprenant les étapes consistant à:
fournir une culture comprenant une souche *Yersinia enterocolitica* ATCC 27729, dans laquelle *Yersinia enterocolitica* a été cultivée à 37 °C dans un milieu TSB comprenant 160 µM de 2,2'-dipyridyle ;
inactiver *Yersinia enterocolitica* pour produire une composition comprenant des cellules *Yersinia enterocolitica* inactivées, dans laquelle l'inactivation a lieu dans des conditions qui ne perturbent pas les cellules ; et
recueillir les cellules inactivées.

4. Composition pouvant être obtenue par un procédé comprenant les étapes consistant à:
fournir une culture comprenant une souche *Yersinia pestis* KIM6+, dans laquelle *Yersinia pestis* a été cultivée à 37 °C dans un milieu TSB comprenant 160 µM de 2,2'-dipyridyle ;
perturber *Yersinia pestis* pour produire un mélange comprenant des membranes cellulaires perturbées ;
solubiliser le mélange en ajoutant au mélange un détergent biologique pour produire une préparation comprenant des protéines solubilisées et non solubilisées ; et
isoler les polypeptides non solubilisés.

5. Composition comprenant une préparation de cellules entières isolées d'une souche *Yersinia pestis* KIM6+, dans laquelle les cellules ont été cultivées à 37 °C dans un milieu TSB comprenant 160 µM de 2,2'-dipyridyle.

6. Composition pouvant être obtenue par un procédé comprenant les étapes consistant à:
fournir une culture comprenant une souche *Yersinia pestis* KIM6+, dans laquelle *Yersinia pestis* a été cultivée à 37 °C dans un milieu TSB comprenant 160 µM de 2,2'-dipyridyle ;
inactiver *Yersinia pestis* pour produire une composition comprenant des cellules *Yersinia pestis* inactivées, dans laquelle l'inactivation a lieu dans des conditions qui ne perturbent pas les cellules ; et
recueillir les cellules inactivées.

7. Composition selon l'une quelconque des revendications 1 à 6 pour une utilisation en tant que médicament.
